(19) Europäisches Patentamt
European Patent Office
Office européen des brevets

(11) Veröffentlichungsnummer: **0 048 363**
**B1**

(12) # EUROPÄISCHE PATENTSCHRIFT

(45) Veröffentlichungstag der Patentschrift: **31.07.85**

(21) Anmeldenummer: **81106900.4**

(22) Anmeldetag: **03.09.81**

(51) Int. Cl.[4]: **C 07 D 401/12**, C 07 D 233/02, A 61 K 31/44 // (C07D401/12, 233:02, 213:89),(C07D401/12, 233:02, 213:30)

(54) **Imidazolderivate, Verfahren und Zwischenprodukte zu ihrer Herstellung sowie diese enthaltende Arzneimittel.**

(30) Priorität: **10.09.80 CH 6798/80**
**24.06.81 CH 4175/81**

(43) Veröffentlichungstag der Anmeldung: **31.03.82 Patentblatt 82/13**

(45) Bekanntmachung des Hinweises auf die Patenterteilung: **31.07.85 Patentblatt 85/31**

(84) Benannte Vertragsstaaten: **AT BE CH DE FR GB IT LI LU NL SE**

(56) Entgegenhaltungen: **DE - A - 2 756 269**

(73) Patentinhaber: **F. HOFFMANN-LA ROCHE & CO. Aktiengesellschaft, CH-4002 Basel (CH)**

(72) Erfinder: **Ramuz, Henri, Prof. Dr., Rheinparkstrasse 3, CH-4127 Birsfelden (DE)**

(74) Vertreter: **Lederer, Franz, Dr. et al, Patentanwälte Dr. Lederer Franz Meyer-Roxlau Reiner F. Lucile-Grahn-Strasse 22, D-8000 München 80 (DE)**

EP 0 048 363 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Imidazolderivate der allgemeinen Formel

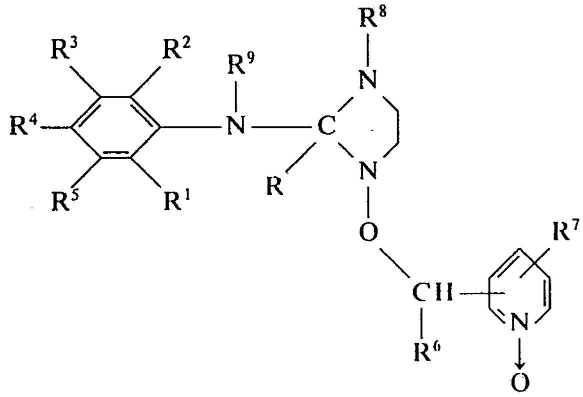

(I)

worin $R^1$, $R^2$ und $R^3$ unabhängig voneinander Wasserstoff, Halogen, Trifluormethyl, Nitro, niederes Alkyl oder niederes Alkylthio, einer der Reste $R^4$ und $R^5$ Wasserstoff und der andere Wasserstoff, Halogen, Hydroxy oder niederes Alkoxy, $R^6$ Wasserstoff oder niederes Alkyl und $R^7$ Wasserstoff, Hydroxy, niederes Alkyl oder niederes Alkoxy bedeuten, und entweder $R^8$ Wasserstoff, niederes Alkyl, niederes Alkenyl, Phenyl-niederes Alkyl oder Acyl und $R^9$ zusammen mit R eine zusätzliche Bindung bedeuten, oder $R^9$ niederes Alkyl, niederes Alkenyl oder Phenyl-niederes Alkyl und $R^8$ zusammen mit R eine zusätzliche Bindung bedeuten, mit der Maßgabe, daß $R^4$ oder $R^5$ nicht Hydroxy bedeutet, wenn $R^7$ niederes Alkoxy und/oder $R^8$ Acyl bedeuten, und daß $R^7$ nicht Hydroxy bedeutet, wenn $R^4$ oder $R^5$ niederes Alkoxy und/oder $R^8$ Acyl bedeuten, und pharmazeutisch annehmbare Säureadditionssalze davon.

Diese Verbindungen sind neu und zeichnen sich durch unerwartete wertvolle pharmakologische Eigenschaften aus.

Gegenstand der vorliegenden Erfindung sind Imidazolderivate der obigen allgemeinen Formel I und ihre pharmazeutisch annehmbaren Säureadditionssalze als solche und als pharmazeutische Wirkstoffe, die Herstellung dieser Verbindungen und Zwischenprodukte für die Herstellung dieser Verbindungen, ferner Arzneimittel, enthaltend eine oder mehrere Verbindungen der allgemeinen Formel I oder pharmazeutisch annehmbare Säureadditionssalze davon und die Herstellung solcher Arzneimittel.

Verbindungen der allgemeinen Formel I, worin $R^6$ niederes Alkyl bedeutet, können in der (R)- oder in der (S)-Konfiguration oder als Gemische dieser beiden Konfigurationen vorliegen. Die Erfindung umfaßt sowohl die reinen optischen Isomeren als auch Mischungen davon, insbesondere die entsprechenden Racemate.

Der Ausdruck »niederes Alkyl«, für sich allein genommen oder in Kombinationen, wie »niederes Alkylthio«, »niederes Alkoxy« und dergleichen, bezeichnet geradkettige oder verzweigte gesättigte Kohlenwasserstoffreste mit höchstens 6, vorzugsweise höchstens 4 Kohlenstoffatomen, wie Methyl, Äthyl, n-Propyl, Isopropyl, Isobutyl, n-Butyl, t-Butyl, Amyl, Hexyl usw. Der Ausdruck »niederes Alkylthio« umfaßt Reste wie Methylthio, Äthylthio, n-Propylthio, Isopropylthio und dergleichen. Der Ausdruck »niederes Alkoxy« umfaßt Reste wie Methoxy, Äthoxy, n-Propoxy, Isopropoxy und dergleichen. Der Ausdruck »Halogen« bedeutet Fluor, Chlor, Brom oder Jod. Der Ausdruck »niederes Alkenyl« umfaßt geradkettige oder verzweigte Kohlenwasserstoffreste mit 2 bis 6 Kohlenstoffatomen, worin mindestens eine Kohlenstoff-Kohlenstoffbindung ungesättigt ist, wie Allyl, Butenyl, Isobutenyl und dergleichen. Der Ausdruck »Phenyl-niederes Alkyl« umfaßt Reste, wie Benzyl, Phenäthyl und dergleichen. Der Ausdruck »Acyl« umfaßt Benzoyl, Anisoyl-niedere Alkanoyl- und Phenyl-niedere Alkanoylreste, wie Formyl, Acetyl, Propionyl, Butyryl, Phenylacetyl und dergleichen.

$R^1$, $R^2$ und $R^3$ bedeuten vorzugsweise unabhängig voneinander Wasserstoff, Fluor, Chlor, Brom, Trifluormethyl, Methyl, Äthyl oder Isopropyl, $R^4$ oder $R^5$ bedeutet vorzugsweise Wasserstoff, Chlor, Hydroxy oder Methoxy. $R^6$ bedeutet vorzugsweise Wasserstoff oder Methyl. $R^7$ bedeutet vorzugsweise Wasserstoff, Hydroxy, Methyl oder Methoxy. $R^8$ bedeutet vorzugsweise Wasserstoff, wobei $R^9$ zusammen mit R eine zusätzliche Bindung bedeutet.

Eine bevorzugte Klasse von Verbindungen der allgemeinen Formel I umfaßt solche, worin $R^1$ oder $R^3$ Wasserstoff bedeutet und $R^2$ und $R^3$ bzw. $R^1$ und $R^2$ die gleiche Bedeutung besitzen, wobei $R^2$ und $R^3$ bzw. $R^1$ und $R^2$ vorzugsweise Halogen und besonders bevorzugt Chlor bedeuten.

Eine ganz besonders bevorzugte Klasse von Verbindungen der allgemeinen Formel I umfaßt solche, worin entweder $R^1$ und $R^2$ beide Chlor und $R^3$ Wasserstoff, oder $R^2$ und $R^3$ beide Chlor und $R^1$ Wasserstoff bedeuten, $R^4$, $R^5$ und $R^8$ Wasserstoff und $R^9$ zusammen mit R eine zusätzliche Bindung bedeuten.

Eine im Rahmen der vorliegenden Erfindung ganz besonders bevorzugte Verbindung ist

2-[[[2-[(2,6-Dichlorphenyl)imino]-1-imidazolidinyl]oxy]methyl]pyridin-1-oxyd.

Weitere im Rahmen der vorliegenden Erfindung besonders bevorzugte Verbindungen sind:

2-[[[2-[(2,3-Dichlorphenyl)imino]-1-imidazolidinyl]oxy]methyl]pyridin-1-oxyd;
2-[[[2-(2,6-Dichlor-N-methylanilino)-2-imidazolin-1-yl]oxy]methyl]pyridin-1-oxyd;
2-[[[2-[(2,6-Dichlorphenyl)imino]-1-imidazolidinyl]oxy]methyl]-4-methylpyridin-1-oxyd;
4-[1-[[2-[(2,6-Dichlorphenyl)imino]-1-imidazolidinyl]oxy]äthyl]pyridin-1-oxyd;
2-[[[2-[(2,6-Dichlorphenyl)imino]-1-imidazolidinyl]oxy]methyl]-5-methylpyridin-1-oxyd;
2-[[[2-[(2,6-Dichlorphenyl)imino]-1-imidazolidinyl]oxy]methyl]-6-methylpyridin-1-oxyd und
2-[[[2-[(2,6-Dichlorphenyl)imino]-1-imidazolidinyl]oxy]methyl]-4-pyridinol-1-oxyd.

Die neuen Verbindungen der obigen allgemeinen Formel I und deren pharmazeutisch annehmbare Säureadditionssalze können erfindungsgemäß hergestellt werden, indem man

a) eine Verbindung der allgemeinen Formel

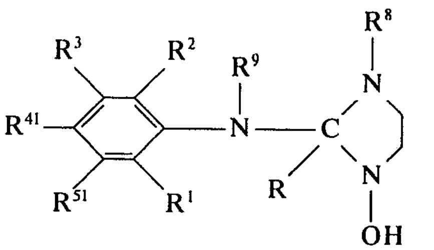

(II)

worin einer der Reste $R^{41}$ und $R^{51}$ Wasserstoff und der andere Wasserstoff, Halogen oder niederes Alkoxy bedeutet, und $R^1$, $R^2$, $R^3$, $R^8$, $R^9$ und R obige Bedeutung besitzen, mit einer Verbindung der allgemeinen Formel

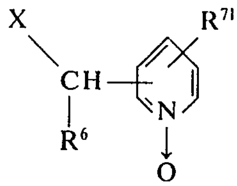

(III)

worin X eine Abgangsgruppe und $R^{71}$ Wasserstoff, niederes Alkyl oder niederes Alkoxy bedeuten, und $R^6$ obige Bedeutung besitzt, umsetzt, oder

b) eine Verbindung der allgemeinen Formel

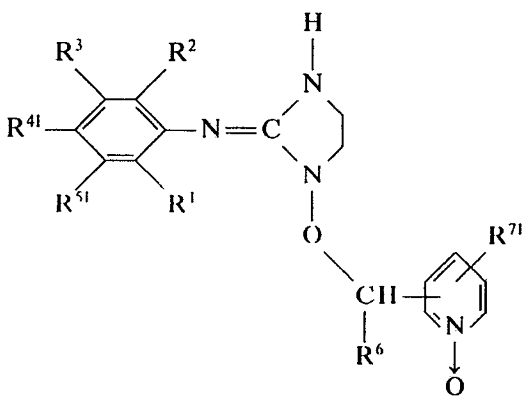

(Ia)

worin $R^1$, $R^2$, $R^3$, $R^{41}$, $R^{51}$, $R^6$ und $R^{71}$ obige Bedeutung besitzen, am sekundären Stickstoffatom acyliert, oder

c) eine Verbindung der allgemeinen Formel

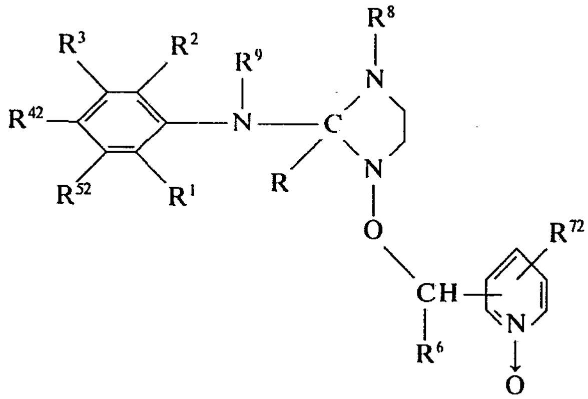

(Ib)

worin einer der Reste $R^{42}$ und $R^{52}$ Wasserstoff und der andere niederes Alkoxy und $R^{72}$ Wasserstoff, niederes Alkyl oder niederes Alkoxy bedeuten, oder $R^{72}$ niederes Alkoxy und einer der Reste $R^{42}$ und $R^{52}$ Wasserstoff und der andere Wasserstoff, Halogen oder niederes Alkoxy bedeuten, und $R^1$, $R^2$, $R^3$, $R^6$, $R^8$, $R^9$ und R obige Bedeutung besitzen,
für die Ätherspaltung geeigneten Bedingungen unterwirft, oder

d) eine Verbindung der allgemeinen Formel

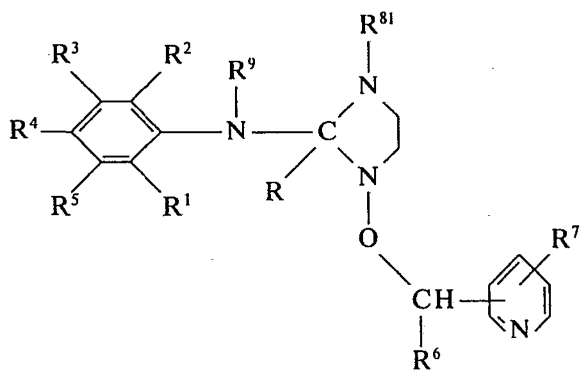

(IV)

worin entweder $R^{81}$ niederes Alkyl, niederes Alkenyl, Phenyl-niederes Alkyl oder Acyl und $R^9$ zusammen mit R eine zusätzliche Bindung bedeuten, oder $R^9$ niederes Alkyl, niederes Alkenyl oder Phenyl-niederes Alkyl und $R^{81}$ zusammen mit R eine zusätzliche Bindung bedeuten, und $R^1$, $R^2$, $R^3$, $R^4$, $R^5$, $R^6$ und $R^7$ obige Bedeutung besitzen, mit der Maßgabe, daß $R^4$ oder $R^5$ nicht Hydroxy bedeutet, wenn $R^7$ niederes Alkoxy und/oder $R^{81}$ Acyl bedeuten, und daß $R^7$ nicht Hydroxy bedeutet, wenn $R^4$ oder $R^5$ niederes Alkoxy und/oder $R^{81}$ Acyl bedeuten,
am Pyridin-Stickstoffatom oxydiert, oder

e) in einer Verbindung der allgemeinen Formel

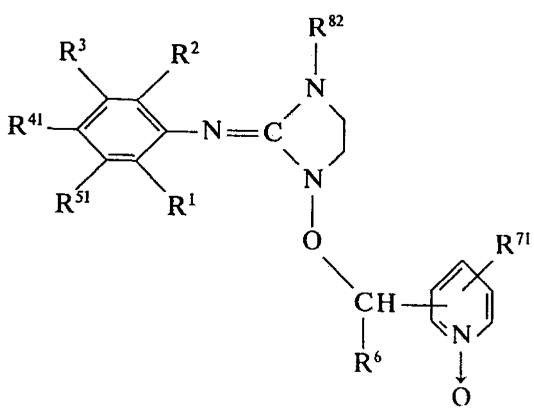

(Ic)

worin $R^{82}$ Acyl bedeutet, und $R^1$, $R^2$, $R^3$, $R^{41}$, $R^{51}$, $R^6$ und $R^{71}$ obige Bedeutung besitzen,
die Acylgruppe entfernt, und

f) erwünschtenfalls ein erhaltenes Gemisch der optischen Antipoden auftrennt, und
g) erwünschtenfalls eine erhaltene Verbindung der allgemeinen Formel I in ein pharmazeutisch annehmbares Säureadditionssalz überführt.

Die Reaktion einer Verbindung der allgemeinen Formel II mit einer Verbindung der allgemeinen Formel III, worin die mit X bezeichnete Abgangsgruppe an sich bekannte Gruppen, wie Halogen, beispielsweise Chlor, Brom oder Jod, Arylsulfonyloxy, wie p-Toluolsulfonyloxy, Alkylsulfonyloxy, wie Methansulfonyloxy, und quaternäre Ammonium- und Sulfoniumsalze umfaßt, kann nach an sich bekannten Methoden durchgeführt werden. Beispielsweise kann man eine Verbindung der Formel II in einem Zweiphasensystem in Gegenwart eines Phasentransfer-Katalysators und einer Base mit einer Verbindung der Formel III umsetzen. Geeignete Zweiphasensysteme sind beispielsweise Wasser/Toluol, Wasser/Benzol und dergleichen. Als Basen verwendet man vorzugsweise Natriumhydroxid, Natrium- oder Kaliumcarbonat, oder dergleichen. Geeignete Phasentransfer-Katalysatoren sind in erster Linie quaternäre Ammoniumsalze, wie Tetra-n-butyl-ammonium-sulfat, -hydrogensulfat, -hydroxid und dergleichen. Aus Zweckmäßigkeitsgründen arbeitet man vorzugsweise bei Raumtemperatur; man kann aber ohne weiteres auch oberhalb oder unterhalb davon arbeiten.

Die Reaktion einer Verbindung der Formel II mit einer solchen der Formel III kann aber auch in einem inerten organischen Lösungsmittel, wie Dimethylformamid, Dimethylsulfoxid, Tetrahydrofuran, Diäthyläther, t-Butylmethyläther, Toluol, Xylol und dergleichen, in Gegenwart einer Base, wie Natriumhydrid, Kalium-t-butylat und dergleichen, in einem Temperaturbereich von etwa —50°C bis 100°C, vorzugsweise zwischen etwa 20°C und 45°C, durchgeführt werden.

Die Acylierung einer Verbindung der allgemeinen Formel Ia, gemäß Verfahrensvariante b), kann mit jedem geeigneten Acylierungsmittel, z. B. mit einem Carbonsäureanhydrid, wie Acetanhydrid, Benzoesäureanhydrid oder dergleichen, nach an sich bekannten und jedem Fachmann geläufigen Methoden durchgeführt werden.

Die Umwandlung der niederen Alkoxygruppe(n) in einer Verbindung der allgemeinen Formel Ib in die Hydroxygruppe(n), gemäß Verfahrensvariante c), erfolgt ebenfalls nach an sich bekannten Methoden. Die gewünschte Ätherspaltung erfolgt beispielsweise mit Chlor-, Brom- oder Jodwasserstoffsäure in wäßriger oder essigsaurer Lösung bei erhöhter Temperatur, vorzugsweise bei der Siedetemperatur des Reaktionsgemisches, oder mit Bortribromid, Bortrichlorid oder dergleichen in einem inerten organischen Lösungsmittel, wie Pentan, Benzol, Toluol, Methylenchlorid, usw., in einem Temperaturbereich von etwa 0°C bis Raumtemperatur.

Eine allenfalls im Molekül vorhandene Acylgruppe wird unter den oben beschriebenen Bedingungen ebenfalls abgespalten.

Die Oxidation einer Verbindung der allgemeinen Formel IV am Pyridin-Stickstoffatom, gemäß Verfahrensvariante d), kann mit verschiedenen an sich bekannten Oxidationsmitteln durchgeführt werden. Als Oxidationsmittel eignen sich in erster Linie Persäuren, wie Perbenzoesäure, m-Chlorperbenzoesäure, Peressigsäure oder dergleichen, Wasserstoffperoxid oder Alkylhydroperoxide, wie t-Butylhydroperoxid. Geeignete Lösungsmittel sind, je nach verwendetem Oxidationsmittel, Alkohole, wie Methanol, halogenierte Kohlenwasserstoffe, wie Methylenchlorid und Chloroform, niedere Fettsäuren, wie Ameisensäure, Essigsäure und Propionsäure, Kohlenwasserstoffe, wie Benzol, Toluol, Cyclohexan und Pentan, oder dergleichen.

Die Entfernung der Acylgruppe in einer Verbindung der allgemeinen Formel Ic, gemäß Verfahrensvariante e), wird vorzugsweise mit einer wäßrigen Säure, wie Salzsäure, Bromwasserstoffsäure, verdünnte Schwefelsäure oder dergleichen, gegebenenfalls in Gegenwart eines Lösungsvermittlers, wie Methanol, Äthanol, Tetrahydrofuran, Dioxan oder der gleichen, durchgeführt. Je nach verwendeter Säure kann man in einem Temperaturbereich von etwa 0°C bis Siedetemperatur des Reaktionsgemisches arbeiten.

Die Auftrennung eines erhaltenen Gemisches der optischen Antipoden, gemäß Verfahrensvariante f), erfolgt nach an sich bekannten und jedem Fachmann geläufigen Methoden. Solche Trennungen können beispielsweise über entsprechende Säureadditionssalze mit optisch aktiven Säuren durchgeführt werden.

Die Verbindungen der obigen Formel I können in pharmazeutisch annehmbare Säureadditionssalze übergeführt werden, beispielsweise durch Behandeln mit einer anorganischen Säure, wie einer Halogenwasserstoffsäure, z. B. Chlorwasserstoff- oder Bromwasserstoffsäure, Schwefelsäure, Phosphorsäure und dergleichen, oder mit einer organischen Säure wie Weinsäure, Zitronensäure, Methansulfonsäure, Cyclohexylaminosulfonsäure und dergleichen. Ein pharmazeutisch nicht annehmbares Säureadditionssalz einer Verbindung der Formel I kann in an sich bekannter Weise, z. B. durch Behandeln mit Alkali, in die freie Base übergeführt werden und diese, erwünschtenfalls, in ein pharmazeutisch annehmbares Säureadditionssalz umgewandelt werden.

Die als Ausgangsstoffe verwendeten Verbindungen der allgemeinen Formel II, worin $R^8$ Wasserstoff und $R^9$ zusammen mit R eine zusätzliche Bindung bedeuten, gehören einer an sich bekannten Stoffklasse an. Spezifisch nicht vorbeschriebene Vertreter können in Analogie zu den bekannten Verbindungen hergestellt werden. Verschiedene der weiter unten folgenden Beispiele enthalten detaillierte Angaben betreffend die Herstellung solcher Verbindungen.

Verbindungen der allgemeinen Formel II, worin einer der Reste $R^8$ und $R^9$ niederes Alkyl, niederes Alkenyl oder Phenyl-niederes Alkyl bedeutet und der andere zusammen mit R eine zusätzliche Bindung bedeutet, sind neu und können hergestellt werden, indem man eine Verbindung der allgemeinen Formel

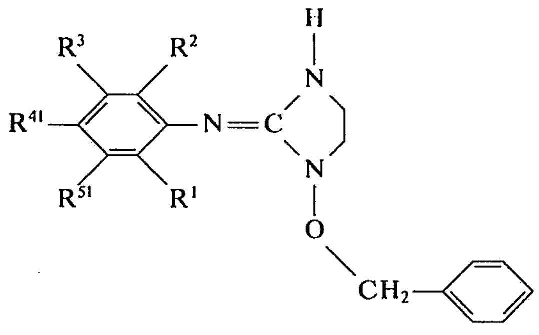

(V)

worin $R^1$, $R^2$, $R^3$, $R^{41}$ und $R^{51}$ obige Bedeutung besitzen, mit einer Verbindung der allgemeinen Formel

$R^9-X$ (VI) bzw. $R^{83}-X$ (VII)

worin $R^{83}$ niederes Alkyl, niederes Alkenyl oder Phenyl-niederes Alkyl bedeutet und $R^9$ und X obige Bedeutung besitzen, umsetzt. Durch geeignete Wahl der Reaktionsbedingungen ist es ohne weiteres möglich, entweder das exo- oder das endocyclische Stickstoffatom selektiv zu alkylieren.

Arbeitet man unter neutralen oder schwach basischen Bedingungen, beispielsweise mit Natrium- oder Kaliumcarbonat in einem inerten organischen Lösungsmittel, wie Methanol, Äthanol, Dimethylformamid, Tetrahydrofuran, Dioxan oder dergleichen, oder in einem Zweiphasensystem in Gegenwart eines Phasentransfer-Katalysators, gegebenenfalls unter Zusatz einer schwachen Base, so kann man mit einer Verbindung der Formel VI selektiv das exocyclische Stickstoffatom alkylieren.

Behandelt man hingegen eine Verbindung der allgemeinen Formel V mit einer starken Base, wie Natriumhydrid, in einem inerten Lösungsmittel, wie Dimethylformamid, Tetrahydrofuran, Dioxan oder dergleichen, so erhält man das entsprechende Anion, das durch eine Verbindung der allgemeinen Formel VII am endocyclischen Stickstoffatom alkyliert wird.

Eine so erhaltene Verbindung entspricht der allgemeinen Formel

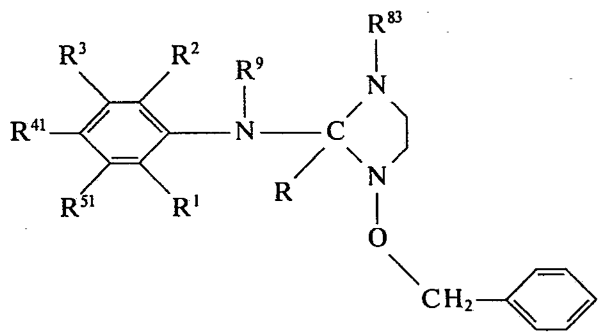

(VIII)

worin einer der Reste $R^{83}$ und $R^9$ niederes Alkyl, niederes Alkenyl oder Phenyl-niederes Alkyl bedeutet, und der andere zusammen mit R eine zusätzliche Bindung bedeutet, und $R^1$, $R^2$, $R^3$, $R^{41}$ und $R^{51}$ obige Bedeutung besitzen.

Aus einer Verbindung der obigen Formel VIII kann man durch Behandeln mit einer Halogenwasserstoffsäure, vorzugsweise Bromwasserstoffsäure, oder mit einer Lewis-Säure, wie Bortrichlorid, in einem inerten Lösungsmittel, wie Methylenchlorid, bei Temperaturen von etwa $-60°C$ bis $0°C$, oder mit Wasserstoff in Gegenwart eines Katalysators, wie Platinoxid, Palladium oder Palladium/Kohle, in einem Lösungsmittel, wie Äthanol, Methanol, Essigsäure, Wasser, Mischungen davon, gegebenenfalls unter Zusatz einer Mineralsäure, wie Salzsäure, die Benzylgruppe abspalten. Es ist selbstverständlich, daß nur solche Reaktionsbedingungen angewendet werden dürfen, die andere im Molekül vorhandene Strukturelemente nicht in unerwünschter Weise beeinträchtigen. Man erhält eine Verbindung der allgemeinen Formel

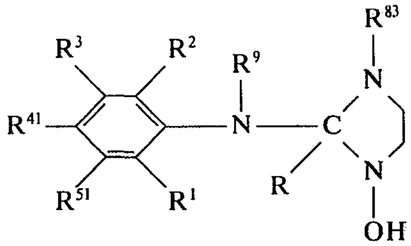

(IIa)

worin $R^1$, $R^2$, $R^3$, $R^{41}$, $R^{51}$, $R^{83}$, $R^9$ und R obige Bedeutung besitzen.

Verbindungen der Formel II, worin $R^8$ Acyl bedeutet, können hergestellt werden, indem man in einer Verbindung der Formel V die Benzylgruppe wie oben beschrieben entfernt und eine so erhaltene Verbindung der allgemeinen Formel

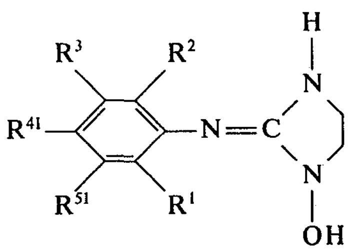

(IX)

worin $R^1$, $R^2$, $R^3$, $R^{41}$ und $R^{51}$ obige Bedeutung besitzen, am sekundären Stickstoffatom acyliert. Diese Acylierung kann mit jedem geeigneten Acylierungsmittel, beispielsweise mit einem Anhydrid (z. B. Acetanhydrid, Benzoesäureanhydrid) oder einem Säurechlorid (z. B. Acetylchlorid, Phenylessigsäurechlorid), durchgeführt werden. Die geeigneten Reaktionsbedingungen können dabei von jedem Fachmann leicht ermittelt werden.

Die Verbindungen der allgemeinen Formel V gehören einer an sich bekannten Stoffklasse an. Spezifisch nicht vorbeschriebene Vertreter können in an sich bekannter Weise, d. h. in zur Herstellung der bekannten Vertreter analoger Weise, hergestellt werden.

Die als Ausgangsstoffe verwendeten Verbindungen der allgemeinen Formel IV können beispielsweise ausgehend von Verbindungen der allgemeinen Formel

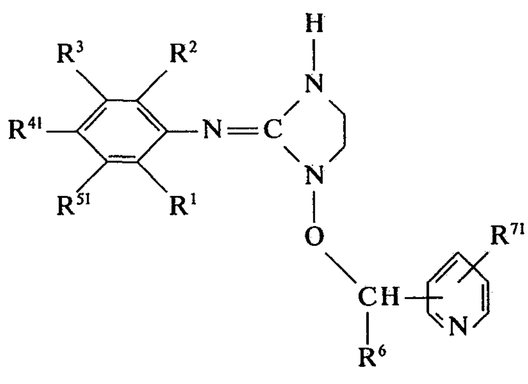

(X)

worin $R^1$, $R^2$, $R^3$, $R^{41}$, $R^{51}$, $R^6$ und $R^{71}$ obige Bedeutung besitzen, welche an sich bekannt oder nach bekannten Methoden herstellbar sind, hergestellt werden. Beispielsweise kann man eine Verbindung der Formel X mit einem reaktiven Derivat einer Carbonsäure, z. B. mit einem Anhydrid, wie Acetanhydrid, Benzoesäureanhydrid und dergleichen, oder mit einem Säurechlorid, wie Acetylchlorid und Benzoylchlorid, oder dergleichen, oder mit Ameisensäure und Acetanhydrid umsetzen. Man erhält dabei eine Verbindung der allgemeinen Formel

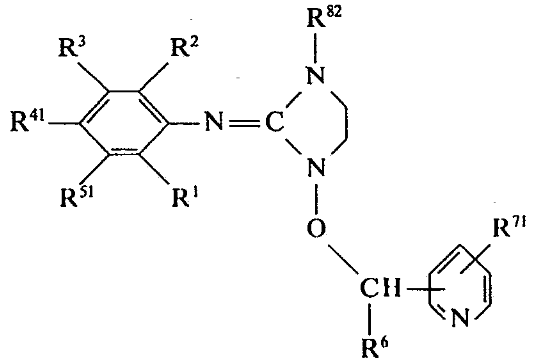

(IVa)

worin $R^1$, $R^2$, $R^3$, $R^{41}$, $R^{51}$, $R^6$, $R^{71}$ und $R^{82}$ obige Bedeutung besitzen.

Weiterhin ist es möglich, eine Verbindung der Formel IIa, in Analogie zu Verfahrensvariante a), mit einer Verbindung der Formel

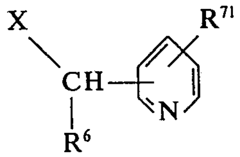

(XI)

worin X, $R^6$ und $R^{71}$ obige Bedeutung besitzen, umzusetzen, wobei man eine Verbindung der allgemeinen Formel

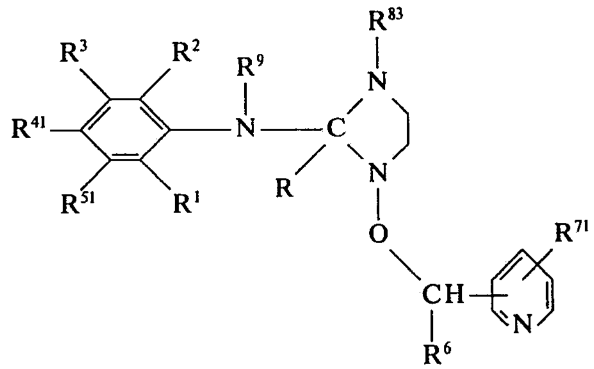

(IVb)

worin $R^1$, $R^2$, $R^3$, $R^{41}$, $R^{51}$, $R^6$, $R^{71}$, $R^{83}$, $R^9$ und R obige Bedeutung besitzen, erhält.

Verbindungen der allgemeinen Formel IV, worin $R^4$ oder $R^5$ und/oder $R^7$ Hydroxy bedeuten, können aus Verbindungen der allgemeinen Formel IVb, worin $R^{41}$ oder $R^{51}$ und/oder $R^{71}$ niederes Alkoxy bedeuten, erhalten werden; und zwar in Analogie zu Verfahrensvariante c).

Die als Ausgangsstoffe verwendeten Verbindungen der allgemeinen Formel II, worin $R^8$ nicht Wasserstoff bedeutet, und der allgemeinen Formel IV sind neu und ebenfalls Gegenstand der vorliegenden Erfindung.

Die erfindungsgemäßen Imidazolderivate der allgemeinen Formel I zeichnen sich wie eingangs erwähnt durch wertvolle pharmakologische Eigenschaften aus. Insbesondere sind sie analgetisch wirksam und können demgemäß als Analgetica bei der Bekämpfung bzw. Verhütung von Schmerzen verwendet werden.

Die analgetischen Eigenschaften von Verbindungen der allgemeinen Formel I können mit dem nachstehend beschriebenen Writhing-Test ermittelt werden:

Zur Durchführung der Versuche werden jeweils 8 männliche Mäuse (20—22 g) pro Dosis eingesetzt. 60 Minuten nach erfolgter oraler Verabreichung der Testsubstanz wird den Tieren 10 ml/kg der Testlösung intraperitoneal injiziert. Anschließend an eine Latenzzeit von 5 Minuten registriert man die Zahl der Tiere, bei denen während 5 Minuten nicht mehr als 1 charakteristisches Writhing-Symptom (konvulsive Streckbewegung des Körpers) auftritt. Die ED 50 gibt diejenige Dosis in mg/kg (p. o.) an, bei welcher 50% der Tiere nicht mehr als 1 Writhing zeigen.

A 2-[[[2-[(2,6-Dichlorphenyl)imino]-1-imidazolidinyl]oxy]methyl]pyridin-1-oxyd
B 2-[[[2 [(2,3-Dichlorphenyl)imino]-1-imidazolidinyl]oxy]methyl]pyridin-1-oxyd
C 2-[[[2-(2,6-Dichlor-N-methylanilino)-2-imidazolin 1-yl]oxy]methyl]pyridin-1-oxyd
D 2-[[[2-[(2,6-Dichlorphenyl)imino]-1-imidazolidinyl]oxy]methyl]-4-methylpyridin-1-oxyd
E 4-[1-[[2-[(2,6-Dichlorphenyl)imino]-1-imidazolidinyl]oxy]äthyl]pyridin-1-oxyd
F 2-[[[2-[(2,6-Dichlorphenyl)imino]-1-imidazolidinyl]oxy]methyl]-5-methylpyridin-1-oxyd
G 2-[[[2-[(2,6-Dichlorphenyl)imino]-1-imidazolidinyl]oxy]methyl]-6-methylpyridin-1-oxyd
H 2-[[[2-[(2,6-Dichlorphenyl)imino]-1-imidazolidinyl]oxy]methyl]-4-pyridinol-1-oxyd.

Writhing-Test (Maus)

60 Minuten nach peroraler Gabe

| | $ED_{50}$ in mg/kg (p.o.) |
|---|---|
| A | 3,5 |
| B | 1,3 |
| C | 26,0 |
| D | 16,0 |
| E | 7,3 |
| F | 27,0 |
| G | 16,0 |
| H | 8,7 |

Die Verbindung A bewirkt an Affen, nach peroraler Gabe, eine dreimal stärkere Wirkung gegen eine elektrische Stimulierung der Zahnpulpa als Morphin. Dagegen konnte keine morphin-agonistische Aktivität gefunden werden, woraus auf die Abwesenheit einer suchtmachenden Wirkung geschlossen werden kann. Die durch die Verbindung A verursachte Analgesie läßt sich mit dem $\alpha$-Adrenorezeptorenblocker Yohimbin, aber nicht mit dem Opiatrezeptorenblocker Naloxon aufheben. Diese Tatsache weist mit aller Deutlichkeit darauf hin, daß diese Analgesie über einen von Opiatrezeptoren unabhängigen Mechanismus hervorgerufen wird.

Überraschenderweise hat es sich gezeigt, daß die erfindungsgemäßen Imidazolderivate der allgemeinen Formel I, im Gegensatz zu anderen pharmazeutisch wirksamen Imidazolderivaten keine, oder höchstens minimale, kardiovaskuläre Wirkungen, und keine zentrale sympathico-inhibitorische Wirkung besitzen.

Die kardiovaskulären Wirkungen können nach der folgenden Methode bestimmt werden:

Der systolische Blutdruck und die Herzfrequenz werden vor Substanzgabe mehrmals an wachen, spontan hypertonen weiblichen Ratten gemessen. Pro Dosis werden jeweils 5 Versuchstiere mit einem Körpergewicht von ca. 300 g eingesetzt. Die Substanzgabe erfolgt mittels Magensonde. Beide Parameter werden 1, 3, 6 und 16 Stunden nach der Applikation gemessen und die prozentuale Veränderung zu den Kontrollwerten berechnet. Der systolische Blutdruck wird indirekt an der Schwanzarterie der Ratte nach der Methode von Gerold et al. gemessen (Helv. Physiol. Acta 24, 58—69 [1966]; Arzneimittelforschung 18, 1285—1287 [1968]).

Die zentrale sympathico-inhibitorische Wirkung kann nach der folgenden Methode bestimmt werden:

Die Wirkung der Testverbindungen auf die Aktivität im sympathischen Nervensystem wird an Katzen in Urethannarkose untersucht. Die präganglionäre Sympathicusaktivität wird mittels bipolaren Platinelektroden vom Nervus splanchnicus, die postganglionäre von einem Nervenast zur Niere nach der Methode von G. Häusler abgeleitet (Naunyn-Schmiedeberg's Arch. Pharmacol. 286, 97—111 [1974]). Außerdem wird der arterielle Blutdruck aus der arteria femoralis sowie die Herzfrequenz gemessen. Die Testsubstanz wird i. v. injiziert. Wenn eine Testsubstanz in blutdrucksenkenden Dosen die Sympathicusaktivität während mehr als 30 Minuten um mehr als 30% hemmt, so wird sie als »mit zentraler symphatico-inhibitorischer Wirkung« qualifiziert.

In der nachfolgenden Tabelle sind die mit den Verbindungen A bis H erhaltenen Resultate zusammengestellt, wobei die maximalen prozentualen Abweichungen von den Kontrollwerten angegeben sind.

| Verbindung | Anaesthetisierte Katze Dosis in mg/kg (i.v.) | Blutdruck, Δ% | Herzfrequenz, Δ% | Zentrale sympathicoinhibitorische Wirkung | Spontan hypertensive Ratte Dosis in mg/kg (p.o.) | Blutdruck, Δ% | Herzfrequenz, Δ% |
|---|---|---|---|---|---|---|---|
| A | 1,0 | − 5 | − 5 | nein | 1,0 | − 0,2 | +15,2 |
| | 3,0 | −10 | − 5 | | 3,0 | + 0,3 | + 3,3 |
| | 10,0 | −10 | −29 | | 10,0 | − 3,1 | − 9,6 |
| | | | | | 30,0 | −10,5 | − 8,6 |
| | | | | | 100,0 | 0 | 0 |
| B | 1,0 | − 5 | 0 | nein | 1,0 | − 4,1 | −11,8 |
| | 3,0 | −25 | − 6 | | 3,0 | − 7,8 | − 8,4 |
| | 10,0 | −50 (15′) | − 6 | | | | |
| C | 1,0 | −14 (5′) | − 6 | nein | 1,0 | + 0,3 | + 6,3 |
| | 3,0 | −21 (10′) | −22 (10′) | | 3,0 | + 1,2 | − 5,8 |
| | 10,0 | −21 (10′) | −22 (10′) | | 10,0 | + 2,2 | + 3,2 |
| D | 1,0 | −22 (3′) | − 9 | nein | 1,0 | − 7,0 | −10,0 |
| | 3,0 | −22 | −27 | | 3,0 | −10,2 | − 7,5 |
| | | | | | 10,0 | −12,6 | −13,3 |
| E | 1,0 | 0 | 0 | nein | 3,0 | +10,2 | + 4,0 |
| | 3,0 | − 4 | 0 | | 10,0 | −13,9 | −13,2 |
| | 10,0 | −12 | −14 | | 30,0 | + 2,4 | − 9,9 |
| F | 1,0 | 0 | 0 | nein | 1,0 | − 9,6 | − 4,9 |
| | 3,0 | 0 | 0 | | 3,0 | −10,4 | − 5,5 |
| | 10,0 | 0 | −10 | | | | |
| G | 1,0 | − 4 | 0 | nein | 10,0 | −12,0 | −22,6 |
| | 3,0 | − 4 | − 4 | | | | |
| | 10,0 | −15 | −13 | | | | |
| H | 1,0 | −12 | 0 | nein | — | — | — |
| | 3,0 | −27 (10′) | − 2 | | | | |
| | 10,0 | −62 (10′) | −18 (10′) | | | | |

Die Verbindungen der allgemeinen Formel I und ihre pharmazeutisch annehmbaren Säureadditionssalze können als Heilmittel z. B. in Form pharmazeutischer Präparate Verwendung finden, welche sie oder ihre Salze in Mischung mit einem für die enterale oder parenterale Applikation geeigneten pharmazeutischen, organischen oder anorganischen inerten Trägermaterial, wie z. B. Wasser, Gelatine, Gummi arabicum, Milchzucker, Stärke, Magnesiumstearat, Talk, pflanzliche Öle, Polyalkylenglykole, Vaseline, usw. enthalten. Die pharmazeutischen Präparate können in fester Form, z. B. als Tabletten, Dragées, Suppositorien, Kapseln oder in flüssiger Form, z. B. als Lösungen, Suspensionen oder Emulsionen, vorliegen. Gegebenenfalls sind sie sterilisiert und bzw. oder enthalten Hilfsstoffe, wie Konservierungs-, Stabilisierungs-, Netz- oder Emulgiermittel, Salze, zur Veränderung des osmotischen Druckes, oder Puffer. Sie können auch noch andere therapeutisch wertvolle Stoffe enthalten.

Die tägliche Dosis bei oraler Verabreichung liegt zwischen etwa 1 und 200 mg; bei intravenöser Verabreichung zwischen etwa 0,1 und 20 mg. Die angegebenen Dosierungen sind jedoch nur als Beispiele zu verstehen und können je nach der Schwere des zu behandelnden Falles und nach Ermessen des behandelnden Arztes abgeändert werden.

Wie eingangs erwähnt sind Arzneimittel, enthaltend eine Verbindung der allgemeinen Formel I oder ein pharmazeutisch annehmbares Säureadditionssalz davon, ebenfalls Gegenstand der vorliegenden Erfindung, weiterhin auch ein Verfahren zur Herstellung solcher Arzneimittel, welches dadurch gekennzeichnet ist, daß man eine oder mehrere Verbindungen der allgemeinen Formel I oder pharmazeutisch annehmbare Säureadditionssalze davon und gegebenenfalls einen oder mehrere andere therapeutisch wertvolle Stoffe in eine galenische Darreichungsform bringt.

Die nachfolgenden Beispiele erläutern die Erfindung. Alle Temperaturen sind in Celsiusgraden angegeben. Die Schmelzpunkte sind nicht korrigiert.

## Beispiel 1

Eine Suspension von 9,84 g (40 mMol) 2-[(2,6-Dichlorphenyl)imino]-1-hydroxyimidazolidin, 1,4 g (4,1 mMol) Tetra-n-butylammoniumsulfat, 7,56 g (42 mMol) 2-Chlormethylpyridin-N-oxyd-hydrochlorid in 140 ml Toluol wird unter starkem Rühren mit 30 ml 28-proz. Natronlauge versetzt. Die Temperatur steigt sofort bis auf 30°. Nach 2 Stunden wird der Niederschlag abgenutscht, mit Wasser gewaschen und im Vakuum bei 60° getrocknet. Aus Methanol und Acetonitril erhält man 2-[[[2-[(2,6-Dichlorphenyl)imino]-1-imidazolidinyl]oxy]methyl]-pyridin-1-oxyd vom Schmelzpunkt 185—187°. Das entsprechende Hydrochlorid kristallisiert aus Methanol/Acetonitril mit 2,5 Mol HCl und schmilzt bei 171—173°; das entsprechende Cyclohexylsulfamat zersetzt sich langsam von 159—238°.

## Beispiel 2

In Analogie zu den Angaben in Beispiel 1 werden die folgenden Verbindungen hergestellt:

— aus 2-[(2,6-Dichlorphenyl)imino]-1-hydroxyimidazolidin und 3-Chlormethyl-pyridin-N-oxyd erhält man 3-[[[2-[(2,6-Dichlorphenyl)imino]-1-imidazolidinyl]oxy]methyl]pyridin-1-oxyd-dihydochlorid vom Schmelzpunkt 199—200° (Methanol/Aceton),
— aus 2-[(o-Chlorphenyl)imino]-1-hydroxyimidazolidin und 2-Chlormethyl-pyridin-N-oxyd erhält man 2-[[[2-[(o-Chlorphenyl)imino]-1-imidazolidinyl]oxy]methyl]pyridin-1-oxyd-hydrobromid vom Schmelzpunkt 166—167° (Aceton/Methanol),
— aus 2-[(2,6-Dichlorphenyl)imino]-1-hydroxyimidazolidin und 3-[1-Chloräthyl]-pyridin-N-oxyd erhält man 3-[1-[[2-[(2,6-Dichlorphenyl)imino]-1-imidazolidinyl]oxy]äthyl]pyridin-1-oxyd-dihydrobromid vom Schmelzpunkt 271—272° (Aceton/30-proz. Bromwasserstoff in Eisessig),
— aus 1-Hydroxy-2-(phenylimino)imidazolin und 2-Chlormethyl-pyridin-N-oxyd erhält man 2-[[[2-(Phenylimino)-1-imidazolidinyl]oxy]methyl]pyridin-1-oxyd-dihydrobromid vom Schmelzpunkt 173° (Aceton/30-proz. Bromwasserstoff in Eisessig) unter Zersetzung,
— aus 2-[(2,6-Dichlorphenyl)imino]-1-hydroxyimidazolidin und 4-(1-Chloräthyl)-pyridin-N-oxyd erhält man 4-[1-[[2-[(2,6-Dichlorphenyl)imino]-1-imidazolidinyl]oxy]äthyl]pyridin-1-oxyd-dihydrobromid vom Schmelzpunkt 232—233°,
— aus 2-[(2,6-Dichlorphenyl)imino]-1-hydroxyimidazolidin und 4-Methyl-2-chlormethyl-pyridin-N-oxyd erhält man 2-[[[2-[(2,6-Dichlorphenyl)imino]-1-imidazolidinyl]oxy]methyl]-4-methyl-pyridin-1-oxyd vom Schmelzpunkt 181° (Methanol/Acetonitril),
— aus 2-[(2,6-Dichlorphenyl)imino]-1-hydroxyimidazolidin und 5-Methyl-2-chlormethyl-pyridin-N-oxyd erhält man 2-[[[2-[(2,6-Dichlorphenyl)imino]-1-imidazolidinyl]oxy]methyl]-5-methyl-pyridin-1-oxyd vom Schmelzpunkt 201—202° (Acetonitril),
— aus 2-[(2,6-Dichlorphenyl)imino]-1-hydroxyimidazolidin und 2-(1-Chloräthyl)pyridin-N-oxyd erhält man 2-[1-[[2-(2,6-Dichlorphenyl)imino]-1-imidazolidinyl]oxy]äthyl]pyridin-1-oxyd-dihydrobromid vom Schmelzpunkt 176—177° (Aceton/Methanol),

— aus 1-Hydroxy-2-[(α,α,α-trifluor-m-tolyl)imino]imidazolidin und 2-Chlormethyl-pyridin-N-oxyd erhält man 2-[[[2-[(α,α,α-Trifluor-m-tolyl)imino]-1-imidazolidinyl]oxy]methyl]-pyridin-1-oxyd vom Schmelzpunkt 95—97° (Isopropyläther),
— aus 2-[(2,3-Dichlorphenyl)imino]-1-hydroxyimidazolidin und 2-Chlormethyl-pyridin-N-oxyd erhält man 2-[[[2-[(2,3-Dichlorphenyl)imino]-1-imidazolidinyl]oxy]methyl]pyridin-1-oxyd vom Schmelzpunkt 168—169° (Acetonitril),
— aus 1-Hydroxy-2-[(α,α,α-trifluor-o-tolyl)imino]imidazolidin und 2-Chlormethyl-pyridin-N-oxyd erhält man 2-[[[2-[(α,α,α-Trifluor-o-tolyl)imino]-1-imidazolidinyl]oxy]methyl]pyridin-1-oxyd vom Schmelzpunkt 175—176° (Methanol/Aceton),
— aus 2-(o-Cumenylimino)-1-hydroxyimidazolidin und 2-Chlormethyl-pyridin-N-oxyd erhält man 2-[[[2-(o-Cumenylimino)-1-imidazolidinyl]oxy]methyl]pyridin-1-oxyd vom Schmelzpunkt 136—137° (Aceton/Isopropyläther),
— aus 2-[(2,5-Dichlorphenyl)imino]-1-hydroxyimidazolidin und 2-Chlormethyl-pyridin-N-oxyd erhält man 2-[[[2-[(2,5-Dichlorphenyl)imino]-1-imidazolidinyl]oxy]methyl]pyridin-1-oxyd vom Schmelzpunkt 167—168° (Acetonitril),
— aus 1-Hydroxy-2-(2,6-xylylimino)imidazolidin und 2-Chlormethyl-pyridin-N-oxyd erhält man 2-[[[2-[(2,6-Xylylimino)-1-imidazolidinyl]oxy]methyl]pyridin-1-oxyd vom Schmelzpunkt 118—120° (Äther),
— aus 2-[(2,4-Dichlorphenyl)imino]-1-hydroxyimidazolidin und 2-Chlormethyl-pyridin-N-oxyd erhält man 2-[[[2-[(2,4-Dichlorphenyl)imino]-1-imidazolidinyl]oxy]methyl]pyridin-1-oxyd vom Schmelzpunkt 168—169° (Chloroform/Äthanol),
— aus 2-[(2,6-Dibromphenyl)imino]-1-hydroxyimidazolidin und 2-Chlormethyl-pyridin-N-oxyd erhält man 2-[[[2-[(2,6-Dibromphenyl)imino]-1-imidazolidinyl]oxy]methyl]pyridin-1-oxyd vom Schmelzpunkt 163—165° (Äthanol).

## Beispiel 3

a) Eine Lösung von 19,68 g (115 mMol) 2,6-Difluorphenylisothiocyanat und 34,1 g (115 mMol) N-[2-(N-Benzyloxy)amino)äthyl]phthalimid in 150 ml Benzol wird während 6 Stunden unter Rückfluß zum Sieden erhitzt. Die abgekühlte Lösung wird von einem geringen Niederschlag abgetrennt und dann im Vakuum eingedampft. Das zurückbleibende Öl kristallisiert aus Isopropyläther. Man erhält 1-(Benzyloxy)-3-(2,6-difluorphenyl)-1-(2-phthalimidoäthyl)-2-thioharnstoff vom Schmelzpunkt 141—143° (Äther/Methylenchlorid).

b) 30,1 g (64,4 mMol) des so erhaltenen Stoffes werden über Nacht mit 300 ml einer 40-proz. Methylaminlösung gerührt. Anschließend wird die Lösung während einer Stunde bei 50° gehalten, auf 10° abgekühlt und mit Äther extrahiert. Die organische Phase wird dreimal mit einer 15-proz. Weinsäurelösung ausgeschüttelt. Der wäßrige Auszug wird anschließend mit konzentriertem Ammoniak basisch gestellt und mit Äther extrahiert. Die ätherischen Auszüge werden getrocknet und im Vakuum eingedampft. Das zurückbleibende Öl wird ohne weitere Reinigung für die nächste Stufe verwendet. Das 1-(Benzyloxy)-2-[(2,6-difluorphenyl)imino]imidazolidin-hydrochlorid schmilzt bei 159—160° (Aceton).

c) 16,0 g (52,7 mMol) des obigen Stoffes werden mit 100 ml 48-proz. Bromwasserstoffsäure auf 150° erwärmt. Nach 30 Minuten wird die Lösung eingeengt und der Rückstand aus Aceton umkristallisiert. Man erhält 2-[(2,6-Difluorphenyl)imino]-1-hydroxyimidazolidin-hydrobromid vom Schmelzpunkt 225° (Zersetzung).

d) Aus 2-[(2,6-Difluorphenyl)imino]-1-hydroxyimidazolidin und 2-Chlormethyl-pyridin-N-oxyd erhält man, in Analogie zu den Angaben in Beispiel 1, 2-[[[2-[(2,6-Difluorphenyl)imino]-1-imidazolidinyl]oxy]methyl]pyridin-1-oxyd-dihydrochlorid vom Schmelzpunkt 188—189° (Aceton/Chlorwasserstoff in Dioxan).

## Beispiel 4

a) In Analogie zu den Angaben in Beispiel 3a erhält man aus 2-Chlor-5-methoxyphenylisothiocyanat und N-[2-(N-(Benzyloxy)amino)äthyl]phthalimid den 1-(Benzyloxy)-3-(2-chlor-5-methoxyphenyl)-1-(2-phthalimidoäthyl)-2-thioharnstoff vom Schmelzpunkt 138—139° (Methylenchlorid/Äther).

b) Aus dem obigen Stoff erhält man in Analogie zu den Angaben in Beispiel 3b das 1-(Benzyloxy)-2-[(2-chlor-5-methoxyphenyl)imino]imidazolidin-hydrochlorid vom Schmelzpunkt 198—200° (Aceton/Chlorwasserstoff in Dioxan).

c) Eine Lösung von 1,84 g (5 mMol) 1-(Benzyloxy)-2-[(2-chlor-5-methoxyphenyl)imino]imidazolidin in 10 ml Äthanol und 0,65 ml 25-proz. Salzsäure wird über Palladium/Kohle hydriert. Der Katalysator wird anschließend abfiltriert und die erhaltene Lösung im Vakuum eingedampft. Man erhält 2-[(2-Chlor-5-methoxyphenyl)imino]-1-hydroxyimidazolidin-hydrochlorid vom Schmelzpunkt 182—184° (Aceton).

d) Aus 2-[(2-Chlor-5-methoxyphenyl)imino]-1-hydroxyimidazolidin und 2-Chlormethyl-pyridin-N-oxyd erhält man, in Analogie zu den Angaben in Beispiel 1, 2-[[[2-[(2-Chlor-5-methoxyphenyl)imi-

no] 1 imidazolidinyl]oxy]methyl]pyridin-1-oxyd-dihydrobromid vom Schmelzpunkt 167—168° (Methanol/Aceton).

Beispiel 5

a) Eine Lösung von 155,4 g (0,747 Mol) 2,4-Dichlor-3-nitrophenol in 2 l Methylenchlorid wird mit 16,9 g Benzyltrimethylammoniumchlorid versetzt. Dazu gibt man eine Lösung von 44,8 g Natronlauge in 1,8 l Wasser und, unter starkem Rühren, 143 ml Dimethylsulfat. Nach einer Stunde wird die organische Phase abgetrennt, mit Wasser gewaschen, getrocknet und im Vakuum eingedampft. Man erhält 2,4-Dichlor-3-nitroanisol vom Schmelzpunkt 97—99° (Hexan).

b) Eine Lösung von 62,4 g (0,28 Mol) 2,4-Dichlor-3-nitroanisol in 750 ml Äthanol wird mit 19 g Raney-Nickel versetzt und während 8 Stunden hydriert. Die erhaltene Lösung wird filtriert und im Vakuum eingedampft. Den Rückstand nimmt man in Methylenchlorid auf und wäscht die Lösung mit einer verdünnten Natriumcarbonatlösung. Nach Trocknen der organischen Phase über Natriumsulfat und Eindampfen im Vakuum erhält man 2,6-Dichlor-3-methoxyanilin vom Siedepunkt 84° (0,1 Torr).

c) 60,7 g (316 mMol) 2,6-Dichlor-3-methoxyanilin werden in 65 ml Toluol gelöst und mit 25 ml Thiophosgen in 50 ml Toluol versetzt. Die erhaltene Lösung wird über Nacht unter Rückfluß zum Sieden erhitzt und anschließend im Vakuum eingedampft. Man erhält 2,6-Dichlor-3-methoxyphenylisothiocyanat vom Siedepunkt 99° (0,13 Torr).

d) 23,4 g (100 mMol) 2,6-Dichlor-3-methoxyphenylisothiocyanat und 29,0 g (97,8 mMol) N-[2-((Benzyloxy)amino)äthyl]phthalimid werden mit 150 ml Toluol während 5 Stunden unter Rückfluß zum Sieden erhitzt. Der erhaltene Niederschlag wird abgenutscht und getrocknet. Man erhält 1-(Benzyloxy)-3-(2,6-dichlor-3-methoxyphenyl)-1-(2-phthalimidoäthyl)-2-thioharnstoff vom Schmelzpunkt 175—176°.

e) 42,0 g (79,2 mMol) 1-(Benzyloxy)-3-(2,6-dichlor-3-methoxyphenyl)-1-(2-phthalimidoäthyl)-2-thioharnstoff und 20,6 g (108 mMol) Triäthyloxoniumtetrafluoroborat werden in 500 ml Methylenchlorid gelöst und 2 Stunden bei Raumtemperatur stehengelassen. Diese Lösung wird mit einer gesättigten Natriumcarbonatlösung und mit Wasser gewaschen, über Natriumsulfat getrocknet und im Vakuum eingedampft. Man erhält 3-(Benzyloxy)-1-(2,6-dichlor-3-methoxyphenyl)-2-äthyl-3-(2-phthalimidoäthyl)-2-thiopseudoharnstoff vom Schmelzpunkt 121—122° (Isopropyläther).

f) Eine Mischung aus 46,0 g (82,4 mMol) 3-(Benzyloxy)-1-(2,6-dichlor-3-methoxyphenyl)-2-äthyl-3-(2-phthalimidoäthyl)-2-thiopseudoharnstoff, 100 ml Äthanol und 300 ml einer 40-proz. Lösung von Methylamin in Wasser wird bei Raumtemperatur über Nacht gerührt. Die Lösung wird 2 Stunden auf 50° erwärmt, abgekühlt und mit Äther extrahiert. Die organische Phase wird getrocknet und im Vakuum eingedampft. Der Rückstand wird in 250 ml Toluol aufgenommen. Die erhaltene Lösung wird über Nacht zum Sieden erhitzt, abgekühlt, mit 3N Natronlauge und mit Wasser gewaschen, über Natriumsulfat getrocknet und im Vakuum eingedampft. Ein Teil des zurückbleibenden Öls wird in Aceton gelöst und mit dioxanischem Chlorwasserstoff versetzt. Man erhält 1-(Benzyloxy)-2-(2,6-dichlor-3-methoxyphenyl)imino-imidazolidin-dihydrochlorid vom Schmelzpunkt 222—223° (Methanol/Acetonitril).

g) Eine Lösung von 20,14 g (50 mMol) 1-(Benzyloxy)-2-(2,6-dichlor-3-methoxyphenyl)imino-imidazolidin in 100 ml Äthanol wird mit 6,5 ml konzentrierter Salzsäure und 1,0 g Palladium/Kohle versetzt und bei Raumtemperatur hydriert. Nach 2 Stunden wird die Lösung filtriert und im Vakuum eingedampft. Das zurückbleibende Material wird in Aceton aufgenommen und unter Rückfluß zum Sieden erhitzt. Man erhält 2-[(2,6-Dichlor-3-methoxyphenyl)imino]-1-hydroxyimidazolidin-hydrochlorid vom Schmelzpunkt 215—217°.

h) 19,0 g (60,7 mMol) 2-[2,6-Dichlor-3-methoxyphenyl)imino]-1-hydroxyimidazolidin-hydrochlorid werden in 160 ml Toluol suspendiert. Dazu gibt man sukzessiv 40 ml einer 40-proz. Lösung von Tetra-n-butylammoniumhydroxyd, 12,2 g (84,7 mMol) 2-Chlormethyl-pyridin-N-oxyd und 250 ml einer gesättigten Kaliumcarbonatlösung. Nach 4 Stunden wird das ausgefallene Material abfiltriert, mit Wasser und anschließend mit Äther gewaschen und aus Chloroform und Äthanol umkristallisiert. Man erhält 2-[[[2-[(2,6-Dichlor-3-methoxyphenyl)imino]-1-imidazolidinyl]oxy]methyl]pyridin-1-oxyd vom Schmelzpunkt 199—200°C. Das entsprechende Dihydrobromid schmilzt unter Zersetzung bei 192° (Methanol/Acetonitril).

Beispiel 6

a) 57,6 g (0,3 Mol) 2,6-Dichlor-4-methoxy-anilin werden portionenweise, innert 10 Minuten, zu einem gerührten, eisgekühlten Gemisch von 27,6 g (0,6 Mol) Ameisensäure und 61,2 g (0,6 Mol) Essigsäureanhydrid gegeben. Das feste Reaktionsgemisch wird mit 50 ml Ameisensäure verdünnt und filtriert. Der Niederschlag wird in Essigester aufgenommen. Die organische Lösung wird sukzessiv mit Eiswasser, 3N Salzsäure und gesättigter Natriumcarbonatlösung gewaschen, getrocknet und im Vakuum eingedampft. Nach Umkristallisieren des Rückstandes aus Essigester erhält man 2,6-Dichlor-4-me-

thoxyformanilid vom Schmelzpunkt 152—153°.

b) Eine Lösung von 59,3 g (0,27 Mol) 2,6-Dichlor-4-methoxyformanilid in 600 ml absolutem Methylenchlorid wird mit 54,6 g (0,54 Mol) Triäthylamin versetzt. Unter Kühlen werden bei 20°, innert 30 Minuten, 200 ml einer 20-proz. Phosgenlösung in Toluol (0,405 Mol) zugetropft. Man rührt noch 30 Minuten bei Raumtemperatur, versetzt die gelbe Suspension mit 600 ml absolutem Methylenchlorid und stellt unter Kühlen mit gasförmigem Ammoniak basisch. Die Suspension wird filtriert und eingedampft. Den Rückstand versetzt man mit 300 ml absolutem Tetrahydrofuran, filtriert und dampft erneut ein. Nach Umkristallisieren aus Methylenchlorid/Hexan erhält man 2,6-Dichlor-4-methoxyphenyl-isocyanid vom Schmelzpunkt 110—112°.

c) Eine Lösung von 44 g (0,218 Mol) 2,6-Dichlor-4-methoxyphenyl-isocyanid in 250 ml trockenem Tetrahydrofuran wird bei 20° über einen Zeitraum von 15 Minuten mit einer Lösung von 83 g (0,22 Mol) Phenyltrimethylammoniumbromid-dibromid versetzt. Nach dem Eindampfen der gelb-roten Lösung erhält man 2,6-Dichlor-N-(dibrommethylen)-p-anisidin als rot-braunes Öl, das ohne weitere Reinigung in die nächste Stufe eingesetzt wird.

d) Eine Aufschlämmung von 52,6 g (0,22 Mol) N-(Benzyloxy)-äthylendiamin-dihydrochlorid in 200 ml Wasser wird portionenweise mit 76 g (0,55 Mol) Kaliumcarbonat versetzt. Unter Kühlen und gutem Rühren tropft man bei 15° eine Lösung von 80 g (0,22 Mol) 2,6-Dichlor-N-(dibrommethylen)-p-anisidin in 100 ml trockenem Tetrahydrofuran zu. Danach wird die Suspension auf Eis gegossen und mit Äther extrahiert. Die organische Phase wird mit einer gesättigten Kochsalzlösung gewaschen, getrocknet und eingedampft. Durch Behandeln des erhaltenen Öls mit Äther erhält man 1-(Benzyloxy)-2-[(2,6-dichlor-4-methoxyphenyl)imino]imidazolin vom Schmelzpunkt 90—92°.

e) 44,3 g (0,12 Mol) 1-(Benzyloxy)-2-[(2,6-dichlor-4-methoxyphenyl)imino]imidazolin werden in 350 ml Äthanol und 350 ml Essigsäure gelöst und mit 13,8 ml (0,12 Mol) Benzylchlorid versetzt. In Gegenwart von 1 g Platinoxid wird unter Normaldruck hydriert. Nach beendeter Wasserstoffaufnahme wird der Katalysator abfiltriert und die Lösung eingedampft. Der Rückstand wird mit Wasser versetzt und mit Äther gewaschen. Die wäßrige Phase wird alkalisch gestellt und mit Essigsäureäthylester ausgeschüttelt. Nach dem Trocknen und Eindampfen der organischen Auszüge und Umkristallisieren des Rückstandes aus Essigester/Methanol erhält man 2-[(2,6-Dichlor-4-methoxyphenyl)imino]-1-hydroxyimidazolidin vom Schmelzpunkt 199—201°.

f) Aus 2-[(2,6-Dichlor-4-methoxyphenyl)imino]-1-hydroxyimidazolidin und 2-Chlormethyl-pyridin-N-oxyd erhält man, in Analogie zu den Angaben in Beispiel 1, 2-[[[2-[(2,6-Dichlor-4-methoxyphenyl)imino]-1-imidazolidinyl]oxy]methyl]pyridin-1-oxyd vom Schmelzpunkt 184—186° (Methanol).

## Beispiel 7

7,3 g (20,9 mMol) 2-[[[2-[(2-Chlor-5-methoxyphenyl)imino]-1-imidazolidinyl]oxy]methyl]pyridin-1-oxyd werden in 150 ml 48-proz. Bromwasserstoffsäure gelöst und während 2 Stunden unter Rückfluß zum Sieden erhitzt. Nach Abkühlen auf Raumtemperatur wird der Niederschlag abgenutscht, mit etwas Äthanol gewaschen und im Vakuum getrocknet. Das 4-Chlor-3-[[1-(2′-pyridylmethoxy)-2-imidazolidinyliden]amino]phenol-1′-oxyd-dihydrobromid schmilzt unter Zersetzung bei 214—216°.

## Beispiel 8

In Analogie zu den Angaben in Beispiel 7 erhält man aus 2-[[[2-(2,6-Dichlor-3-methoxyphenyl)imino]-1-imidazolidinyl]oxy]methyl]pyridin-1-oxyd und 48-proz. Bromwasserstoffsäure 2,4-Dichlor-3-[[1-(2′-pyridylmethoxy)-2-imidazolidinyliden]imino]phenol-1′-oxyd-dihydrobromid vom Schmelzpunkt 196—197° (Zersetzung; Methanol/Aceton).

## Beispiel 9

Aus 2-[[[2-[(2,6-Dichlor-4-methoxyphenyl)imino]-1-imidazolidinyl]oxy]methyl]pyridin-1-oxyd und 48-proz. Bromwasserstoffsäure erhält man, in Analogie zu den Angaben in Beispiel 7, 3,5-Dichlor-4-[[1-(2′-pyridylmethoxy)-2-imidazolidinyliden]imino]phenol-1′-oxyd vom Schmelzpunkt 208—210° (48-proz. Bromwasserstoffsäure).

## Beispiel 10

a) Eine Lösung von 4,2 g (12,4 mMol) 2-[[[2-[(2,6-Dichlorphenyl)imino]-1-imidazolidinyl]oxy]methyl]pyridin in 25 ml Essigsäureanhydrid wird während 2 Stunden auf 55° erwärmt und anschließend im Vakuum eingedampft. Der Rückstand wird in Äther aufgenommen. Die ätherische Phase wird mit

einer gesättigten Natriumbicarbonatlösung ausgeschüttelt, getrocknet und im Vakuum eingedampft. Man erhält 1-Acetyl-2-[(2,6-dichlorphenyl)imino]-3-(2-pyridylmethoxy)imidazolidin vom Schmelzpunkt 104—105° (Isopropyläther).

b) 2,0 g (5,2 mMol) von diesem Material werden in 20 ml Chloroform gelöst und bei Raumtemperatur mit 1,3 g (6,7 mMol) m-Chlorperbenzoesäure versetzt. Nach 2 Stunden wird die Lösung mit einer 5-proz. Natriumcarbonatlösung gewaschen, über Natriumsulfat getrocknet und im Vakuum eingedampft. Der Rückstand wird aus Acetonitril/Methylenchlorid umkristallisiert. Man erhält 1-Acetyl-2-[(2,6-dichlorphenyl)imino]-3-(2′-pyridylmethoxy)imidazolidin-1′-oxyd vom Schmelzpunkt 200—202°.

## Beispiel 11

705 mg (1,78 mMol) 1-Acetyl-2-[(2,6-dichlorphenyl)imino]-3-(2′-pyridylmethoxy)imidazolidin-1′-oxyd und 3 ml 3N Salzsäure werden während 30 Minuten auf 50° erwärmt. Die Lösung wird mit Natronlauge basisch gestellt und mit Essigester extrahiert. Die organischen Auszüge werden getrocknet und im Vakuum eingedampft. Der Rückstand wird aus Acetonitril umkristallisiert. Das erhaltene 2-[[[2-[(2,6-dichlorphenyl)imino]-1-imidazolidinyl]oxy]methyl]pyridin-1-oxyd schmilzt bei 185—186°.

## Beispiel 12

a) 2,3 g (6,8 mMol) 2-[[[2-[(2,5-Dichlorphenyl)imino]-1-imidazolidinyl]oxy]methyl]pyridin und 5,1 ml Ameisensäure werden auf 5° abgekühlt und mit 12,2 ml Essigsäureanhydrid versetzt. Die Lösung wird auf Raumtemperatur erwärmt und im Vakuum eingedampft. Der Rückstand wird in Äther aufgenommen. Die organische Phase mit einer gesättigten Natriumbicarbonatlösung gewaschen, getrocknet und im Vakuum eingedampft. Das zurückbleibende Öl chromatographiert man an Kieselgel mit einem Gemisch aus 4 Teilen Chloroform und 1 Teil Essigester als Elutionsmittel. Man erhält kristallines 2-[(2,5-Dichlorphenyl)imino]-1-formyl-3-(2-pyridylmethoxy)imidazolidin vom Schmelzpunkt 91—92° (Isopropyläther).

b) In Analogie zu den Angaben in Beispiel 10b erhält man aus 2-[(2,5-Dichlorphenyl)imino]-1-formyl-3-(2-pyridylmethoxy)imidazolidin und m-Chlorperbenzoesäure das 2-[(2,5-Dichlorphenyl)imino]-1-formyl-3-(2′-pyridylmethoxy)imidazolidin-1′-oxyd vom Schmelzpunkt 146—148° (Aceton).

## Beispiel 13

Eine Lösung von 0,4 g (1 mMol) 2-[(2,5-Dichlorphenyl)imino]-1-formyl-3-(2′-pyridylmethoxy)imidazolidin-1′-oxyd in 5 ml Äthanol wird mit 3 ml 3N Schwefelsäure versetzt, während 2 Tagen bei Raumtemperatur stehengelassen, mit einer gesättigten Natriumbicarbonatlösung basisch gestellt und mit Methylenchlorid extrahiert. Die organische Lösung wird getrocknet und im Vakuum eingedampft. Das durch Umkristallisieren des Rückstandes aus Acetonitril erhaltene 2-[[[2-[(2,5-Dichlorphenyl)imino]-1-imidazolidinyl]oxy]methyl]pyridin-1-oxyd schmilzt bei 167—168°.

## Beispiel 14

a) In Analogie zu den Angaben in Beispiel 12a erhält man aus 2-[[[2-[(6-Chlor-o-tolyl)imino]-1-imidazolidinyl]oxy]methyl]pyridin, Ameisensäure und Essigsäureanhydrid den 2-[(6-Chlor-o-tolyl)imino]-3-(2-pyridylmethoxy)-1-imidazolidin-carboxaldehyd vom Schmelzpunkt 63—64° (Isopropyläther).

b) Aus 2-[(6-Chlor-o-tolyl)imino]-3-(2-pyridylmethoxy)-1-imidazolidin-carboxaldehyd und m-Chlorperbenzoesäure erhält man, in Analogie zu den Angaben in Beispiel 10b, 2-[(6-Chlor-o-tolyl)imino]-1-formyl-3-(2′-pyridylmethoxy)imidazolidin-1′-oxyd vom Schmelzpunkt 166—167° (Aceton).

## Beispiel 15

Aus 2-[(6-Chlor-o-tolyl)imino]-1-formyl-3-(2′-pyridylmethoxy)imidazolidin-1′-oxyd erhält man, in Analogie zu den Angaben in Beispiel 13, 2-[(6-Chlor-o-tolyl)imino]-3-(2′-pyridylmethoxy)imidazolidin-1′-oxyd-dihydrochlorid vom Schmelzpunkt 171—173° (Dioxan/Aceton).

## Beispiel 16

a) Eine Lösung von 16,8 g (50 mMol) 1-(Benzyloxy)-2-[(2,6-dichlorphenyl)imino]imidazolidin werden in 150 ml trockenem Methanol wird mit 5,83 g (55 mMol) Natriumcarbonat und 7,81 g (55 mMol) Methyljodid versetzt. Unter Rühren wird das Gemisch während 4 Stunden zum Rückfluß erhitzt und dann im Vakuum eingedampft. Der Rückstand wird zwischen Wasser und Methylenchlorid verteilt. Die organische Phase wird getrocknet und eingedampft. Das zurückbleibende Öl chromatographiert man an Kieselgel unter Eluieren mit einem Gemisch aus Chloroform und Äthanol (9 : 1). Man erhält ein Öl, das in Aceton gelöst und mit dioxanischem Chlorwasserstoff und dann mit Äther bis zur Trübung behandelt wird. Das erhaltene 1-(Benzyloxy)-2-(2,6-dichlor-N-methylanilino)-2-imidazolin-hydrochlorid schmilzt bei 185—186°.

b) 12,6 g (36 mMol) der entsprechenden freien Base werden während einer Stunde mit 70 ml 48-proz. Bromwasserstoffsäure auf 150° erhitzt. Zur erkalteten Lösung gibt man etwas Eis und eine Spatelspitze Norit. Die Lösung wird filtriert und mit konzentriertem Ammoniak basisch gestellt. Der dabei entstandene Niederschlag wird abgenutscht, mit Wasser gewaschen und aus Methanol/Acetonitril umkristallisiert. Das 2-(2,6-Dichlor-N-methylanilino)-1-hydroxy-2-imidazolin schmilzt bei 177—178° unter Zersetzung.

c) Aus 2-(2,6-Dichlor-N-methylanilino-1-hydroxy-2-imidazolin und 2-Chlormethyl-pyridin-N-oxyd erhält man, in Analogie zu den Angaben in Beispiel 1, 2-[[[2-(2,6-Dichlor-N-methylanilino)-2-imidazolin-1-yl]oxy]methyl]pyridin-1-oxyd-dihydrochlorid vom Schmelzpunkt 156—157°.

## Beispiel 17

a) 9,9 g (29,4 mMol) 1-(Benzyloxy)-2-[(2,6-dichlorphenyl)imino]imidazolidin, 1,0 g Tetra-n-butylammoniumsulfat und 5,0 ml Allylbromid werden mit 35 ml Wasser während 20 Minuten auf 100° erwärmt. Das erhaltene Gemisch wird mit einer gesättigten Natriumcarbonatlösung versetzt und mit Äther extrahiert. Die organische Phase wird getrocknet und im Vakuum eingedampft. Das zurückbleibende ölige 2-(N-Allyl-2,6-dichloranilino)-1-(benzyloxy)-2-imidazolin wird ohne weitere Reinigung in die nächste Stufe eingesetzt.

b) 9,0 g (24 mMol) 2-(N-Allyl-2,6-dichloranilino)-1-(benzyloxy)-2-imidazolin werden in 200 ml Methylenchlorid gelöst und auf —40° abgekühlt. Dazu tropft man eine Lösung von 14,5 g Bortrichlorid in 200 ml Methylenchlorid, läßt auf Raumtemperatur erwärmen und dampft die Lösung im Vakuum ein. Der Rückstand wird mit einer gesättigten Natriumcarbonatlösung und Methylenchlorid versetzt. Der entstandene Niederschlag wird abfiltriert und aus Methanol umkristallisiert. So erhält man 2-(N-Allyl-2,6-dichloranilino)-1-hydroxy-2-imidazolin vom Schmelzpunkt 204° (Zersetzung).

c) Aus 2-(N-Allyl-2,6-dichloranilino)-1-hydroxy-2-imidazolin und 2-Chlormethyl-pyridin-N-oxyd erhält man, in Analogie zu den Angaben in Beispiel 1, 2-[[[2-(N-Allyl-2,6-dichloranilino)-1-imidazolin-1-yl]oxy]methyl]pyridin-1-oxyd vom Schmelzpunkt 116—117° (Isopropyläther).

## Beispiel 18

a) 16,8 g (50 mMol) 1-(Benzyloxy)-2-[(2,6-dichlorphenyl)imino]-imidazolidin werden in 80 ml Dimethylformamid gelöst und unter Rühren bei Raumtemperatur mit 1,44 g (60 mMol) Natriumhydrid versetzt. Nach einer Stunde tropft man eine Lösung von 4 ml (60 mMol) Methyljodid in 20 ml Toluol dazu, wobei die Temperatur bis auf 48° steigt. Nach 16 Stunden wird das Gemisch auf Eis gegossen und mit Äther extrahiert. Die organische Phase wird nacheinander mit Wasser, 15-proz. Weinsäurelösung und Wasser gewaschen, über Magnesiumsulfat getrocknet und im Vakuum eingedampft. Das erhaltene 1-(Benzyloxy)-2-[(2,6-dichlorphenyl)imino]-3-methylimidazolidin wird ohne weitere Reinigung in die nächste Stufe eingesetzt.

b) 20,6 g (58,8 mMol) 1-(Benzyloxy)-2-[(2,6-dichlorphenyl)imino]-3-methylimidazolidin werden mit 100 ml 48-proz. Bromwasserstoffsäure auf 150° erwärmt. Nach einer Stunde wird die Lösung auf Eis gegossen, mit Norit versetzt und filtriert. Das Filtrat wird mit konzentriertem Ammoniak basisch gestellt. Der entstandene Niederschlag wird abgenutscht, mit Wasser gewaschen und getrocknet. Das 2-[(2,6-Dichlorphenyl)imino]-1-hydroxy-3-methylimidazolidin schmilzt bei 187—189° (Zersetzung).

c) Aus 1-[(2,6-Dichlorphenyl)imino]-1-hydroxy-3-methylimidazolidin und 2-Chlormethyl-pyridin-N-oxyd erhält man, in Analogie zu den Angaben in Beispiel 1, 2-[[[2-[(2,6-Dichlorphenyl)imino]-3-methyl-1-imidazolidinyl]oxy]methyl]pyridin-1-oxyd-dihydrochlorid vom Schmelzpunkt 177—178° (Aceton/Chlorwasserstoff in Dioxan).

## Beispiel 19

a) Eine Suspension von 21,6 g (90 mMol) N-(Benzyloxy)-äthylendiamin-dihydrochlorid in 250 ml Toluol wird mit 18,8 g (100 mMol) o-Tolyl-imidocarbonylchlorid, 3,4 g Tetra-n-butylammoniumhydrogensulfat und, unter starkem Rühren, tropfenweise mit 80 ml 28-proz. Natronlauge versetzt. Die Temperatur steigt dabei bis auf 52°. Das Gemisch wird über Nacht gerührt und mit Äther verdünnt. Die organische Phase wird abgetrennt, mit Wasser gewaschen und dreimal mit 3N Schwefelsäure extrahiert. Die

wässerige saure Phase wird mit konzentrierter Natronlauge alkalisch gestellt und mit Äther extrahiert. Die ätherische Lösung wird getrocknet und im Vakuum eingedampft. Man erhält 1-(Benzyloxy)-2-[(o-tolyl)imino]imidazolidin als dickflüssiges Öl, das direkt in die nächste Stufe eingesetzt wird.

b) 22,5 g (80 mMol) 1-(Benzyloxy)-2-[(o-tolyl)imino]imidazolidin werden während 30 Minuten mit 150 ml 48-proz. Bromwasserstoffsäure auf 150° erwärmt. Das Reaktionsgemisch wird im Vakuum eingedampft. Aus dem Rückstand erhält man 1-Hydroxy-2-(o-tolylimino)imidazolidin-hydrobromid vom Schmelzpunkt 176—187° (Aceton).

c) Aus 1-Hydroxy-2-(o-tolylimino)imidazolidin und 2-Chlormethyl-pyridin-N-oxyd erhält man, in Analogie zu den Angaben in Beispiel 1, 2-[[[2-(o-Tolylimino)-1-imidazolidinyl]oxy]methyl]pyridin-1-oxyd-dihydrobromid vom Schmelzpunkt 195—196° (Methanol/Aceton).

Beispiel 20

a) In Analogie zu den Angaben in Beispiel 19a erhält man aus (2,6-Diäthylphenyl)imidocarbonyl-chlorid und N-(Benzyloxy)äthylendiamin-dihydrochlorid das 1-(Benzyloxy)-2-[(2,6-diäthylphenyl)imino]imidazolidin als Öl.

b) In Analogie zu den Angaben in Beispiel 19b erhält man aus 1-(Benzyloxy)-2-[(2,6-diäthylphenyl)imino]imidazolidin und Bromwasserstoffsäure das 2-[(2,6-Diäthylphenyl)imino]-1-hydroxyimidazolidin-hydrobromid vom Schmelzpunkt 129—130° (Acetonitril/Äther).

c) Aus 2-[(2,6-Diäthylphenyl)imino]-1-hydroxyimidazolidin und 2-Chlormethyl-pyridin-N-oxyd erhält man, in Analogie zu den Angaben in Beispiel 1, 2-[[[2-[(2,6-Diäthylphenyl)imino]-1-imidazolidinyl]oxy]methyl]pyridin-1-oxyd vom Schmelzpunkt 124—125° (Acetonitril).

Beispiel 21

a) Aus 2-[[[2-[(2,6-Dichlorphenyl)imino]-1-imidazolidinyl]oxy]methyl]-6-methylpyridin, Ameisensäure und Essigsäureanhydrid erhält man, in Analogie zu den Angaben in Beispiel 12a, 2-[(2,6-Dichlorphenyl)imino]-3-[(6-methyl-2-pyridyl)methoxy]-1-imidazolidincarboxaldehyd vom Schmelzpunkt 85—86° (Isopropyläther).

b) Aus 2-[(2,6-Dichlorphenyl)imino]-3-[(6-methyl-2-pyridyl)methoxy]-1-imidazolidincarboxaldehyd und m-Chlorperbenzoesäure erhält man, in Analogie zu den Angaben in Beispiel 10b, 2-[(2,6-Dichlorphenyl)imino]-3-[(6′-methyl-2′-pyridyl)methoxy]-1-imidazolidincarboxaldehyd-1′-oxyd vom Schmelzpunkt 159—160° (Aceton).

Beispiel 22

Aus 2-[(2,6-Dichlorphenyl)imino]-1-formyl-3-[2′-(6′-methyl)pyridylmethoxy)imidazolidin-1′-oxyd erhält man, in Analogie zu den Angaben in Beispiel 13, 2-[[[2-[(2,6-Dichlorphenyl)imino]-1-imidazolidinyl]oxy]methyl]-6-methylpyridin-1-oxyd vom Schmelzpunkt 196—197° (Methanol/Aceton).

Beispiel 23

Eine Suspension aus 3,5 g (10 mMol) 2-[[[2-[(2,6-Dichlorphenyl)imino]-1-imidazolidinyl]oxy]methyl]pyridin-1-oxyd und 20 ml Essigsäureanhydrid wird während 10 Stunden bei 55° gerührt. Nach dem Abkühlen werden die ausgefallenen Kristalle abgenutscht und mit Cyclohexan gewaschen. Man erhält 1-Acetyl-2-[(2,6-dichlorphenyl)imino]-3-[2′-pyridylmethoxy)imidazolidin-1′-oxyd vom Schmelzpunkt 200—202° (Methylenchlorid/Acetonitril).

Beispiel 24

Aus 2-[(2,6-Dichlorphenyl)imino]-1-hydroxyimidazolidin und 2-(Chlormethyl)-6-methoxypyridin-1-oxyd erhält man, in Analogie zu den Angaben in Beispiel 1, 2-[[[2-[(2,6-Dichlorphenyl)imino]-1-imidazolidinyl]oxy]methyl]-6-methoxypyridin-1-oxyd vom Schmelzpunkt 184—185° (Acetonitril/Isopropyläther).

Beispiel 25

Aus 2-[(2,6-Dichlorphenyl)imino]-1-hydroxyimidazolidin und 2-(Chlormethyl)-3-methoxypyridin-1-oxyd-hydrochlorid erhält man, in Analogie zu den Angaben in Beispiel 1, 2-[[[2-[(2,6-Dichlorphenyl)imino]-1-imidazolidinyl]oxy]methyl]-3-methoxypyridin-1-oxyd-dihydrobromid vom Schmelzpunkt 156—157° (Zersetzung: Methanol/Aceton).

Beispiel 26

a) In Analogie zu den Angaben in Beispiel 1 erhält man aus 2-[(2,6-Dichlorphenyl)imino]-1-hydroxyimidazolidin und 2-(Chlormethyl)-4-methoxypyridin-hydrochlorid das 2-[[[2-[(2,6-Dichlorphenyl)imino]-1-imidazolidinyl]oxy]methyl]-4-methoxypyridin vom Schmelzpunkt 127—128° (Acetonitril).

b) In Analogie zu den Angaben in Beispiel 12a erhält man aus 2-[[[2-[(2,6-Dichlorphenyl)imino]-1-imidazolidinyl]oxy]methyl]-4-methoxypyridin, Ameisensäure und Essigsäureanhydrid den 2-[(2,6-Dichlorphenyl)imino]-3-[(4-methoxy-2-pyridyl)methoxy]-1-imidazolidin-carboxaldehyd vom Schmelzpunkt 134—135° (Methylenchlorid/Isopropyläther).

c) In Analogie zu den Angaben in Beispiel 10b erhält man aus 2-[(2,6-Dichlorphenyl)imino]-3-[(4-methoxy-2-pyridyl)methoxy]-1-imidazolidin-carboxaldehyd und m-Chlorperbenzoesäure das 2-[(2,6-Dichlorphenyl)imino]-3-[(4′-methoxy-2′-pyridyl)methoxy]-1-imidazolidin-carboxaldehyd-1′-oxyd vom Schmelzpunkt 175—176° (Methylenchlorid/Acetonitril).

Beispiel 27

In Analogie zu den Angaben in Beispiel 13 erhält man aus 2-[(2,6-Dichlorphenyl)imino]-3-[(4′-methoxy-2′-pyridyl)methoxy]-1-imidazolidin-carboxaldehyd-1′-oxyd und 3N-Schwefelsäure das 2-[[[2-[(2,6-Dichlorphenyl)imino]-1-imidazolidinyl]oxy]methyl]-4-methoxypyridin-1-oxyd vom Schmelzpunkt 155—157° (Methylenchlorid/Acetonitril).

Beispiel 28

In Analogie zu den Angaben in Beispiel 7 erhält man aus 2-[[[2-[(2,6-Dichlorphenyl)imino]-1-imidazolidinyl]oxy]methyl]-4-methoxypyridin-1-oxyd und 48-proz. Bromwasserstoffsäure das 2-[[[2-[(2,6-Dichlorphenyl)imino]-1-imidazolidinyl]oxy]methyl]-4-pyridinol-1-oxyd-dihydrochlorid vom Schmelzpunkt 182° (Zersetzung; Aceton/Dioxan/Chlorwasserstoff).

Beispiel 29

In Analogie zu den Angaben in Beispiel 7 erhält man aus 2-[[[2-[(2,6-Dichlorphenyl)imino]-1-imidazolidinyl]oxy]methyl]-6-methoxypyridin-1-oxyd und 48-proz. Bromwasserstoffsäure das 6-[[[2-[(2,6-Dichlorphenyl)imino]-1-imidazolidinyl]oxy]methyl]-2-pyridinol-1-oxyd-hydrobromid vom Schmelzpunkt 156—158° (48-proz. Bromwasserstoffsäure).

Beispiel 30

a) In Analogie zu den Angaben in Beispiel 1 erhält man aus 2-[(2,6-Dichlorphenyl)imino]-1-hydroxyimidazolidin und 2-(Chlormethyl)-5-methoxypyridin-hydrochlorid das 2-[[[2-(2,6-Dichlorphenyl)imino]-1-imidazolidinyl]oxy]methyl]-5-methoxypyridin-dihydrochlorid vom Schmelzpunkt 107—108° (Acetonitril/Isopropyläther).

b) In Analogie zu den Angaben in Beispiel 12a erhält man aus 2-[[[2-(2,6-Dichlorphenyl)imino]-1-imidazolidinyl]oxy]methyl]-5-methoxypyridin, Ameisensäure und Essigsäureanhydrid den 2-[(2,6-Dichlorphenyl)imino]-3-[(5-methoxy-2-pyridyl)methoxy]-1-imidazolidin-carboxaldehyd vom Schmelzpunkt 126—127° (Acetonitril).

c) In Analogie zu den Angaben in Beispiel 10b erhält man aus 2-[(2,6-Dichlorphenyl)imino]-3-[(5-methoxy-2-pyridyl)methoxy]-1-imidazolidin-carboxaldehyd und m-Chlorperbenzoesäure das 2-[(2,6-Dichlorphenyl)imino]-3-[(5′-methoxy-2′-pyridyl)methoxy]-1-imidazolidin-carboxaldehyd-1′-oxyd vom Schmelzpunkt 148—149° (Acetonitril).

## Beispiel 31

In Analogie zu den Angaben in Beispiel 13 erhält man aus 2-[(2,6-Dichlorphenyl)imino]-3-[(5'-methoxy-2'-pyridyl)methoxy]-1-imidazolidin-carboxaldehyd-1'-oxyd und 3N-Schwefelsäure das 2-[[[2-[(2,6-Dichlorphenyl)imino]-1-imidazolidinyl]oxy]methyl]-5-methoxypyridin-1-oxyd-dihydrochlorid vom Schmelzpunkt 171—172° (Zersetzung; Acetonitril).

## Beispiel 32

In Analogie zu den Angaben in Beispiel 7 erhält man aus 2-[[[2-[(2,6-Dichlorphenyl)imino]-1-imidazolidinyl]oxy]methyl]-5-methoxypyridin-1-oxyd-dihydrochlorid und 48-proz. Bromwasserstoffsäure das 6-[[[2-[(2,6-Dichlorphenyl)imino]-1-imidazolidinyl]oxy]methyl]-3-pyridinol-1-oxyd-dihydrobromid vom Schmelzpunkt 232° (Zersetzung; Äthanol/Aceton).

## Beispiel 33

In Analogie zu den Angaben in Beispiel 7 erhält man aus 2-[[[2-[(2,6-Dichlorphenyl)imino]-1-imidazolidinyl]oxy]methyl]-3-methoxypyridin-dihydrobromid und 48-proz. Bromwasserstoffsäure das 2-[[[2-[(2,6-Dichlorphenyl)imino]-1-imidazolidinyl]oxy]methyl]-3-pyridinol-1-oxyd-dihydrobromid vom Schmelzpunkt 193—195° (Zersetzung; Aceton).

## Beispiel A

Herstellung von Lacktabletten nachstehender Zusammensetzung:

| | | | |
|---|---|---|---|
| 2-[[[2-[(2,6-Dichlorphenyl)imino]-1-imidazolidinyl]oxy]methyl]pyridin-1-oxyd-N-cyclohexylsulfamat | | | 5,32 mg |
| Milchzucker pulv. | | | 34,68 mg |
| Maisstärke weiß | | | 59,0 mg |
| Talk | | | 0,5 |
| Magnesiumstearat | | | 0,5 mg |
| | Kerngewicht | | 100,0 mg |
| Lacktrockensubstanz | | ca. | 7,0 mg |
| | Lacktablettengewicht | ca. | 107,0 mg |

Die Mischung des Wirkstoffes mit dem Milchzucker pulv. und einem Teil der Maisstärke wird mit einem Kleister aus einem weiteren Teil der Maisstärke und Wasser befeuchtet, geknetet, granuliert, getrocknet und gesiebt. Dieses Granulat wird mit der restlichen Maisstärke, dem Talk und dem Magnesiumstearat gemischt und zu Kernen à 100 mg verpreßt. Die Kerne werden mittels einer der üblichen Methoden mit ca. 7,0 mg Lacktrockensubstanz lackiert.

**Patentansprüche für die Vertragsstaaten BE, CH, DE, FR, GB, IT, LI, LU, NL, SE**

1. Imidazolderivate der allgemeinen Formel

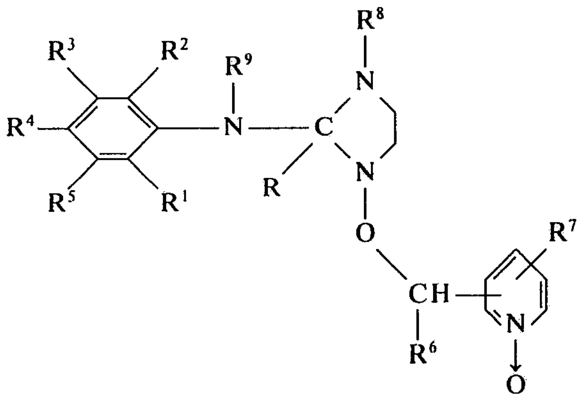

(I)

worin $R^1$, $R^2$ und $R^3$ unabhängig voneinander Wasserstoff, Halogen, Trifluormethyl, Nitro, niederes

Alkyl oder niederes Alkylthio, einer der Reste $R^4$ und $R^5$ Wasserstoff und der andere Wasserstoff, Halogen, Hydroxy oder niederes Alkoxy, $R^6$ Wasserstoff oder niederes Alkyl und $R^7$ Wasserstoff, Hydroxy, niederes Alkyl oder niederes Alkoxy bedeuten, und entweder $R^8$ Wasserstoff, niederes Alkyl, niederes Alkenyl, Phenyl-niederes Alkyl oder Acyl und $R^9$ zusammen mit R eine zusätzliche Bindung bedeuten, oder $R^9$ niederes Alkyl, niederes Alkenyl oder Phenyl-niederes Alkyl und $R^8$ zusammen mit R eine zusätzliche Bindung bedeuten, mit der Maßgabe, daß $R^4$ oder $R^5$ nicht Hydroxy bedeutet, wenn $R^7$ niederes Alkoxy und/oder $R^8$ Acyl bedeuten, und daß $R^7$ nicht Hydroxy bedeutet, wenn $R^4$ oder $R^5$ niederes Alkoxy und/oder $R^8$ Acyl bedeuten, wobei Acyl, niederes Alkanoyl, Benzoyl, Anisoyl oder Phenyl-niederes Alkanoyl bedeutet und die mit niederer bezeichneten Reste höchstens 6 Kohlenstoffatome besitzen, und pharmazeutisch annehmbare Säureadditionssalze davon.

2. Verbindungen gemäß Anspruch 1, dadurch gekennzeichnet, daß $R^7$ Wasserstoff, niederes Alkyl oder niederes Alkoxy bedeutet.

3. Verbindungen gemäß Anspruch 1 oder 2, dadurch gekennzeichnet, daß $R^1$, $R^2$ und $R^3$ unabhängig voneinander Wasserstoff, Fluor, Chlor, Brom, Trifluormethyl, Methyl, Äthyl oder Isopropyl bedeuten.

4. Verbindungen gemäß einem der Ansprüche 1 bis 3, dadurch gekennzeichnet, daß einer der Reste $R^4$ und $R^5$ Wasserstoff und der andere Wasserstoff, Chlor, Hydroxy oder Methoxy bedeutet.

5. Verbindungen gemäß einem der Ansprüche 1 bis 4, dadurch gekennzeichnet, daß $R^6$ Wasserstoff oder Methyl bedeutet.

6. Verbindungen gemäß einem der Ansprüche 1 und 3 bis 5, dadurch gekennzeichnet, daß $R^7$ Wasserstoff, Hydroxy, Methyl oder Methoxy bedeutet.

7. Verbindungen gemäß einem der Ansprüche 1 bis 6, dadurch gekennzeichnet, daß $R^8$ Wasserstoff und $R^9$ zusammen mit R eine zusätzliche Bindung bedeuten.

8. Verbindungen gemäß einem der Ansprüche 1 bis 7, dadurch gekennzeichnet, daß $R^1$ oder $R^3$ Wasserstoff bedeutet und $R^2$ und $R^3$ bzw. $R^1$ und $R^2$ die gleiche Bedeutung besitzen.

9. Verbindungen gemäß Anspruch 8, dadurch gekennzeichnet, daß $R^2$ und $R^3$ bzw. $R^1$ und $R^2$ Halogen bedeuten.

10. Verbindungen gemäß Anspruch 9, dadurch gekennzeichnet, daß $R^2$ und $R^3$ bzw. $R^1$ und $R^2$ Chlor bedeuten.

11. Verbindungen gemäß Anspruch 10, dadurch gekennzeichnet, daß $R^4$, $R^5$ und $R^8$ alle Wasserstoff und $R^9$ zusammen mit R eine zusätzliche Bindung bedeuten.

12. 2-[[[2-[(2,6-Dichlorphenyl)imino]-1-imidazolidinyl]oxy]methyl]pyridin-1-oxyd.

13. 2-[[[2-[(2,3-Dichlorphenyl)imino]-1-imidazolidinyl]oxy]methyl]pyridin-1-oxyd.

14. 2-[[[2-(2,6-Dichlor-N-methylanilino)-2-imidazolin-1-yl]oxy]methyl]pyridin-1-oxyd.

15. 2-[[[2-[(2,6-Dichlorphenyl)imino]-1-imidazolidinyl]oxy]methyl]-4-methylpyridin-1-oxyd.

16. 4-[1-[[2-[(2,6-Dichlorphenyl)imino]-1-imidazolidinyl]oxy]äthyl]pyridin-1-oxyd.

17. 2-[[[2-[(2,6-Dichlorphenyl)imino]-1-imidazolidinyl]oxy]methyl]-5-methylpyridin-1-oxyd.

18. 2-[[[2-[(2,6-Dichlorphenyl)imino]-1-imidazolidinyl]oxy]methyl]-6-methylpyridin-1-oxyd.

19. 2-[[[2-[(2,6-Dichlorphenyl)imino]-1-imidazolidinyl]oxy]methyl]-4-pyridinol-1-oxyd.

20. Verbindungen der allgemeinen Formel

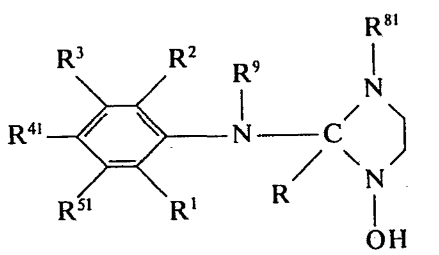

(IIb)

worin $R^1$, $R^2$ und $R^3$ unabhängig voneinander Wasserstoff, Halogen, Trifluormethyl, Nitro, niederes Alkyl oder niederes Alkylthio, einer der Reste $R^{41}$ und $R^{51}$ Wasserstoff und der andere Wasserstoff, Halogen oder niederes Alkoxy, und entweder $R^{81}$ niederes Alkyl, niederes Alkenyl, Phenyl-niederes Alkyl oder Acyl und $R^9$ zusammen mit R eine zusätzliche Bindung, oder $R^9$ niederes Alkyl, niederes Alkenyl oder Phenyl-niederes Alkyl und $R^{81}$ zusammen mit R eine zusätzliche Bindung bedeuten, wobei Acyl, niederes Alkanoyl, Benzoyl, Anisoyl oder Phenyl-niederes Alkanoyl bedeutet und die mit niederer bezeichneten Reste höchstens 6 Kohlenstoffatome besitzen.

21. Verbindungen der allgemeinen Formel

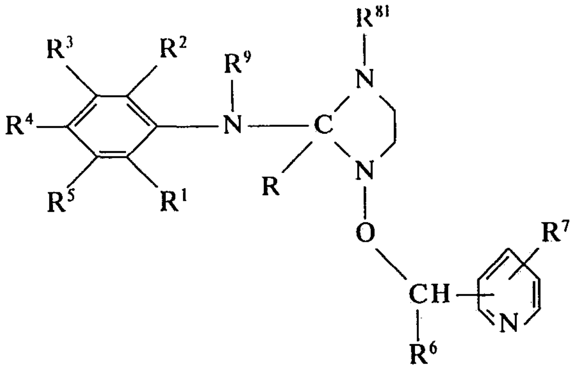

(IV)

worin $R^1$, $R^2$ und $R^3$ unabhängig voneinander Wasserstoff, Halogen, Trifluormethyl, Nitro, niederes Alkyl oder niederes Alkylthio, einer der Reste $R^4$ und $R^5$ Wasserstoff und der andere Wasserstoff, Halogen, Hydroxy oder niederes Alkoxy, $R^6$ Wasserstoff oder niederes Alkyl und $R^7$ Wasserstoff, Hydroxy, niederes Alkyl oder niederes Alkoxy, und entweder $R^{81}$ niederes Alkyl, niederes Alkenyl, Phenyl-niederes Alkyl oder Acyl und $R^9$ zusammen mit R eine zusätzliche Bindung, oder $R^9$ niederes Alkyl, niederes Alkenyl oder Phenyl-niederes Alkyl und $R^{81}$ zusammen mit R eine zusätzliche Bindung bedeuten, mit der Maßgabe, daß $R^4$ oder $R^5$ nicht Hydroxy bedeutet, wenn $R^7$ niederes Alkoxy und/oder $R^8$ Acyl bedeuten, und daß $R^7$ nicht Hydroxy bedeutet, wenn $R^4$ oder $R^5$ niederes Alkoxy und/oder $R^8$ Acyl bedeuten, wobei Acyl, niederes Alkanoyl, Benzoyl, Anisoyl oder Phenyl-niederes Alkanoyl bedeutet und die mit niederer bezeichneten Reste höchstens 6 Kohlenstoffatome besitzen.

22. Verbindungen gemäß einem der Ansprüche 1 bis 11 und 13 bis 19 als pharmazeutische Wirkstoffe.

23. Verbindungen gemäß einem der Ansprüche 1 bis 11 und 13 bis 19 als analgetische Wirkstoffe.

24. 2-[[[2-[(2,6-Dichlorphenyl)imino]-1-imidazolidinyl]oxy]methyl]pyridin-1-oxyd als pharmazeutischer Wirkstoff.

25. 2-[[[2-[(2,6-Dichlorphenyl)imino]-1-imidazolidinyl]oxy]methyl]pyridin-1-oxyd als analgetischer Wirkstoff.

26. Verfahren zur Herstellung von Verbindungen der in Anspruch 1 definierten allgemeinen Formel I und ihrer pharmazeutisch annehmbaren Säureadditionssalze, dadurch gekennzeichnet, daß man

a) eine Verbindung der allgemeinen Formel

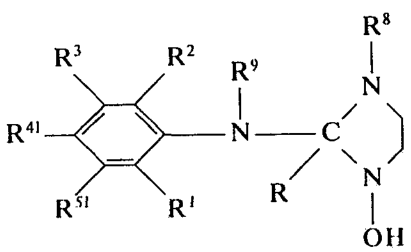

(II)

worin einer der Reste $R^{41}$ und $R^{51}$ Wasserstoff und der andere Wasserstoff, Halogen oder niederes Alkoxy bedeutet, und $R^1$, $R^2$, $R^3$, $R^8$, $R^9$ und R die in Anspruch 1 angegebene Bedeutung besitzen,
mit einer Verbindung der allgemeinen Formel

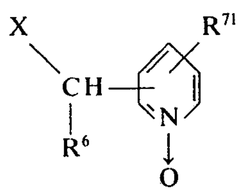

(III)

worin X eine Abgangsgruppe und $R^{71}$ Wasserstoff, niederes Alkyl oder niederes Alkoxy bedeuten, und $R^6$ die in Anspruch 1 angegebene Bedeutung besitzt,
umsetzt, oder

b) eine Verbindung der allgemeinen Formel

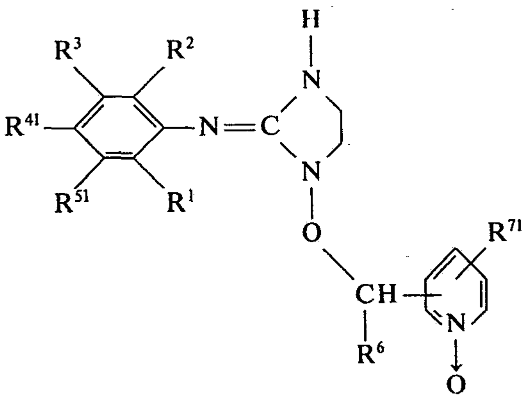

(Ia)

worin $R^1$, $R^2$, $R^3$ und $R^6$ die in Anspruch 1 angegebene und $R^{41}$, $R^{51}$ und $R^{71}$ obige Bedeutung besitzen,
am sekundären Stickstoffatom acyliert, oder

c) eine Verbindung der allgemeinen Formel

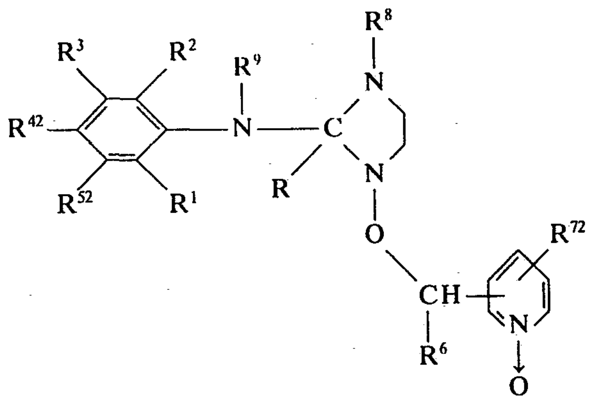

(Ib)

worin einer der Reste $R^{42}$ und $R^{52}$ Wasserstoff und der andere niederes Alkoxy und $R^{72}$ Wasserstoff, niederes Alkyl oder niederes Alkoxy bedeuten, oder $R^{72}$ niederes Alkoxy und einer der Reste $R^{42}$ und $R^{52}$ Wasserstoff und der andere Wasserstoff, Halogen oder niederes Alkoxy bedeuten, und $R^1$, $R^2$, $R^3$, $R^6$, $R^8$, $R^9$ und R die in Anspruch 1 angegebene Bedeutung besitzen,
für die Ätherspaltung geeigneten Bedingungen unterwirft, oder

d) eine Verbindung der allgemeinen Formel

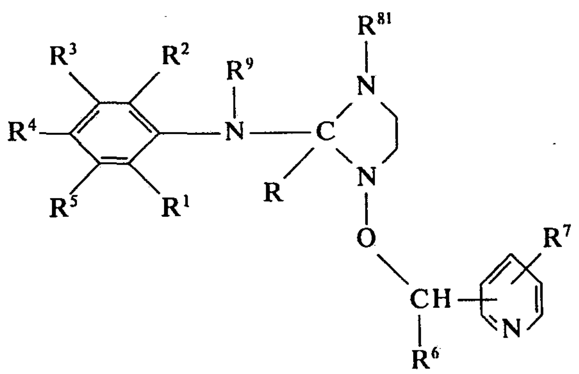

(IV)

worin entweder $R^{81}$ niederes Alkyl, niederes Alkenyl, Phenyl-niederes Alkyl oder Acyl und $R^9$ zusammen mit R eine zusätzliche Bindung bedeuten, oder $R^9$ niederes Alkyl, niederes Alkenyl oder Phenyl-niederes Alkyl und $R^{81}$ zusammen mit R eine zusätzliche Bindung bedeuten, und $R^1$, $R^2$, $R^3$, $R^4$, $R^5$, $R^6$ und $R^7$ die in Anspruch 1 angegebene Bindung besitzen, mit der Maßgabe, daß $R^4$ oder $R^5$ nicht Hydroxy bedeutet, wenn $R^7$ niederes Alkoxy und/oder $R^{81}$ Acyl bedeuten, und daß $R^7$ nicht Hydroxy bedeutet, wenn $R^4$ oder $R^5$ niederes Alkoxy und/oder $R^{81}$ Acyl bedeuten,
am Pyridin-Stickstoffatom oxydiert, oder

e) in einer Verbindung der allgemeinen Formel

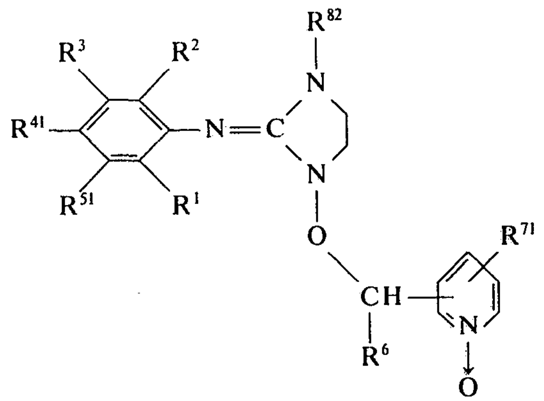

(Ic)

worin $R^{82}$ Acyl bedeutet, und $R^1$, $R^2$, $R^3$ und $R^6$ die in Anspruch 1 angegebene und $R^{41}$, $R^{51}$ und $R^{71}$ obige Bedeutung besitzen, die Acylgruppe entfernt, und

f) erwünschtenfalls ein erhaltenes Gemisch der optischen Antipoden auftrennt, und

g) erwünschtenfalls eine erhaltene Verbindung der allgemeinen Formel I in ein pharmazeutisch annehmbares Säureadditionssalz überführt.

27. Verfahren gemäß Anspruch 26, dadurch gekennzeichnet, daß man 2-[[[2-[(2,6-Dichlorphenyl)imino]-1-imidazolidinyl]oxy]methyl]pyridin-1-oxyd herstellt.

28. Arzneimittel, enthaltend eine Verbindung gemäß einem der Ansprüche 1 bis 11 und 13 bis 19.

29. Analgetika, enthaltend eine Verbindung gemäß einem der Ansprüche 1 bis 11 und 13 bis 19.

30. Arzneimittel, enthaltend 2-[[[2-[(2,6-Dichlorphenyl)imino]-1-imidazolidinyl]oxy]methyl]pyridin-1-oxyd.

31. Analgetika, enthaltend 2-[[[2-[(2,6-Dichlorphenyl)imino]-1-imidazolidinyl]oxy]methyl]pyridin-1-oxyd.

**Patentansprüche für den Vertragsstaat AT**

1. Verfahren zur Herstellung von Imidazolderivaten der allgemeinen Formel

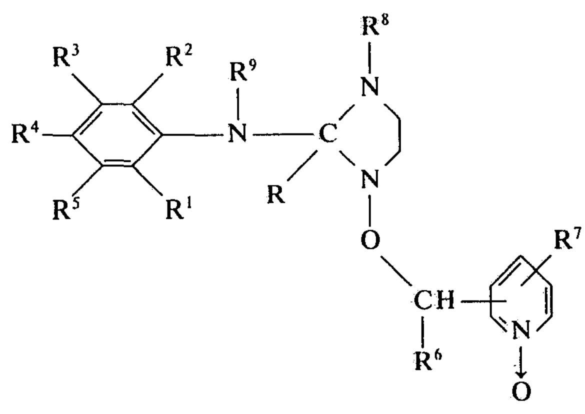

(I)

worin $R^1$, $R^2$ und $R^3$ unabhängig voneinander Wasserstoff, Halogen, Trifluormethyl, Nitro, niederes Alkyl oder niederes Alkylthio, einer der Reste $R^4$ und $R^5$ Wasserstoff und der andere Wasserstoff, Halogen, Hydroxy oder niederes Alkoxy, $R^6$ Wasserstoff oder niederes Alkyl und $R^7$ Wasserstoff, Hydroxy, niederes Alkyl oder niederes Alkoxy bedeuten, und entweder $R^8$ Wasserstoff, niederes Alkyl, niederes Alkenyl, Phenyl-niederes Alkyl oder Acyl und $R^9$ zusammen mit R eine zusätzliche Bindung bedeuten, oder $R^9$ niederes Alkyl, niederes Alkenyl oder Phenyl-niederes Alkyl und $R^8$ zusammen mit R eine zusätzliche Bindung bedeuten, mit der Maßgabe, daß $R^4$ oder $R^5$ nicht Hydroxy bedeutet, wenn $R^7$ niederes Alkoxy und/oder $R^8$ Acyl bedeuten, und daß $R^7$ nicht Hydroxy bedeutet, wenn $R^4$ oder $R^5$ niederes Alkoxy und/oder $R^8$ Acyl bedeuten, wobei Acyl, niederes Alkanoyl, Benzoyl, Anisoyl oder Phenyl-niederes Alkanoyl bedeutet und die mit niederer bezeichneten Reste höchstens 6 Kohlenstoffatome besitzen, und von pharmazeutisch annehmbaren Säureadditionssalzen davon, dadurch gekennzeichnet, daß man

a) eine Verbindung der allgemeinen Formel

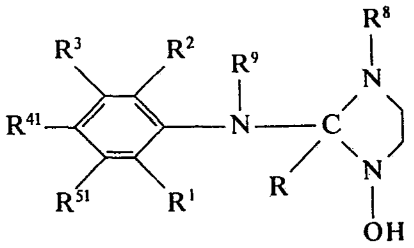

(II)

worin einer der Reste $R^{41}$ und $R^{51}$ Wasserstoff und der andere Wasserstoff, Halogen oder niederes Alkoxy bedeutet, und $R^1$, $R^2$, $R^3$, $R^8$, $R^9$ und R obige Bedeutung besitzen, mit einer Verbindung der allgemeinen Formel

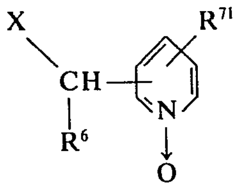

(III)

worin X eine Abgangsgruppe und $R^{71}$ Wasserstoff, niederes Alkyl oder niederes Alkoxy bedeuten, und $R^6$ obige Bedeutung besitzt, umsetzt, oder

b) eine Verbindung der allgemeinen Formel

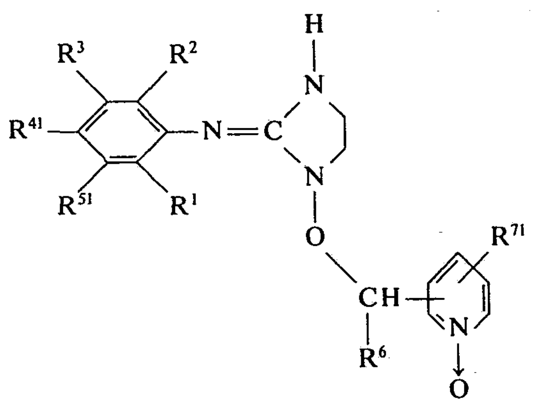

(Ia)

worin $R^1$, $R^2$, $R^3$, $R^{41}$, $R^{51}$, $R^6$ und $R^{71}$ obige Bedeutung besitzen, am sekundären Stickstoffatom acyliert, oder

c) eine Verbindung der allgemeinen Formel

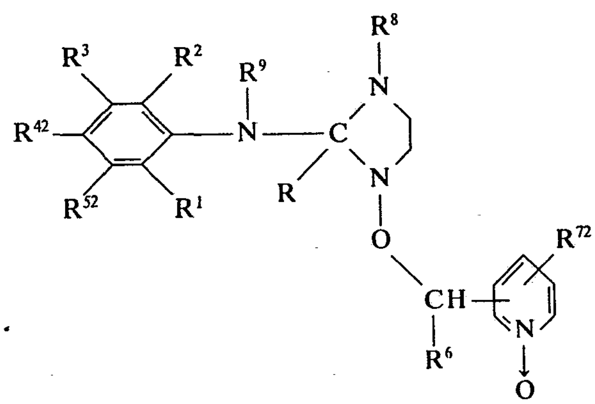

(Ib)

worin einer der Reste $R^{42}$ und $R^{52}$ Wasserstoff und der andere niederes Alkoxy und $R^{72}$ Wasserstoff, niederes Alkyl oder niederes Alkoxy bedeuten, oder $R^{72}$ niederes Alkoxy und einer der Reste $R^{42}$ und $R^{52}$ Wasserstoff und der andere Wasserstoff, Halogen oder niederes Alkoxy bedeuten, und $R^1$, $R^2$, $R^3$, $R^6$, $R^8$, $R^9$ und R obige Bedeutung besitzen,

für die Ätherspaltung geeigneten Bedingungen unterwirft, oder

d) eine Verbindung der allgemeinen Formel

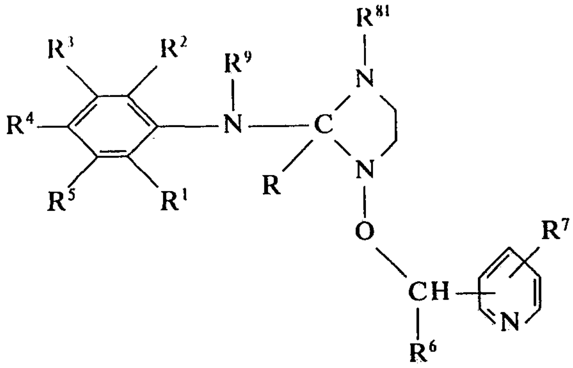

(IV)

worin entweder $R^{81}$ niederes Alkyl, niederes Alkenyl, Phenyl-niederes Alkyl oder Acyl und $R^9$ zusammen mit R eine zusätzliche Bindung bedeuten, oder $R^9$ niederes Alkyl, niederes Alkenyl oder Phenyl-niederes Alkyl und $R^{81}$ zusammen mit R eine zusätzliche Bindung bedeuten, und $R^1$, $R^2$, $R^3$, $R^4$, $R^5$, $R^6$ und $R^7$ obige Bedeutung besitzen, mit der Maßgabe, daß $R^4$ oder $R^5$ nicht Hydroxy bedeutet, wenn $R^7$ niederes Alkoxy und/oder $R^{81}$ Acyl bedeuten, und daß $R^7$ nicht Hydroxy bedeutet, wenn $R^4$ oder $R^5$ niederes Alkoxy und/oder $R^{81}$ Acyl bedeuten,
am Pyridin-Stickstoffatom oxydiert, oder

e) in einer Verbindung der allgemeinen Formel

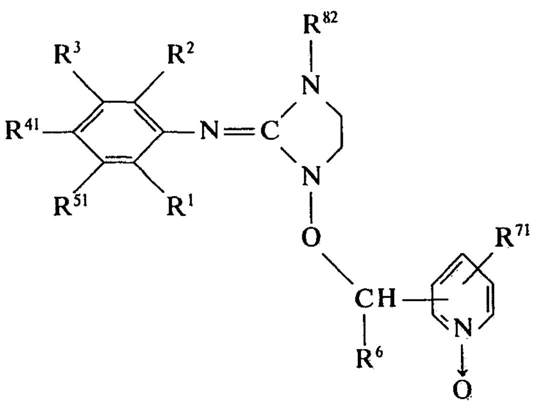

(Ic)

worin $R^{82}$ Acyl bedeutet, und $R^1$, $R^2$, $R^3$, $R^{41}$, $R^{51}$, $R^6$ und $R^{71}$ obige Bedeutung besitzen,
die Acylgruppe entfernt, und

f) erwünschtenfalls ein erhaltenes Gemisch der optischen Antipoden auftrennt, und

g) erwünschtenfalls eine erhaltene Verbindung der allgemeinen Formel I in ein pharmazeutisch annehmbares Säureadditionssalz überführt.

2. Verfahren gemäß Anspruch 1, dadurch gekennzeichnet, daß man Verbindungen der in Anspruch 1 definierten allgemeinen Formel I herstellt, worin $R^7$ Wasserstoff, niederes Alkyl oder niederes Alkoxy bedeutet.

3. Verfahren gemäß Anspruch 1 oder 2, dadurch gekennzeichnet, daß man Verbindungen der in Anspruch 1 definierten allgemeinen Formel I herstellt, worin $R^1$, $R^2$ und $R^3$ unabhängig voneinander Wasserstoff, Fluor, Chlor, Brom, Trifluormethyl, Methyl, Äthyl oder Isopropyl bedeuten.

4. Verfahren gemäß einem der Ansprüche 1 bis 3, dadurch gekennzeichnet, daß man Verbindungen der in Anspruch 1 definierten allgemeinen Formel I herstellt, worin einer der Reste $R^4$ und $R^5$ Wasserstoff und der andere Wasserstoff, Chlor, Hydroxy oder Methoxy bedeutet.

5. Verfahren gemäß einem der Ansprüche 1 bis 4, dadurch gekennzeichnet, daß man Verbindungen der in Anspruch 1 definierten allgemeinen Formel I herstellt, worin $R^6$ Wasserstoff oder Methyl bedeutet.

6. Verfahren gemäß einem der Ansprüche 1 und 3 bis 5, dadurch gekennzeichnet, daß man Verbindungen der in Anspruch 1 definierten allgemeinen Formel I herstellt, worin $R^7$ Wasserstoff, Hydroxy, Methyl oder Methoxy bedeutet.

7. Verfahren gemäß einem der Ansprüche 1 bis 6, dadurch gekennzeichnet, daß man Verbindungen der in Anspruch 1 definierten allgemeinen Formel I herstellt, worin $R^8$ Wasserstoff und $R^9$ zusammen mit R eine zusätzliche Bindung bedeuten.

8. Verfahren gemäß einem der Ansprüche 1 bis 7, dadurch gekennzeichnet, daß man Verbindungen der in Anspruch 1 definierten allgemeinen Formel I herstellt, worin $R^1$ oder $R^3$ Wasserstoff bedeutet und $R^2$ und $R^3$ bzw. $R^1$ und $R^2$ die gleiche Bedeutung besitzen.

9. Verfahren gemäß Anspruch 8, dadurch gekennzeichnet, daß man Verbindungen der in Anspruch 1 definierten allgemeinen Formel I herstellt, worin $R^2$ und $R^3$ bzw. $R^1$ und $R^2$ Halogen bedeuten.

10. Verfahren gemäß Anspruch 9, dadurch gekennzeichnet, daß man Verbindungen der in Anspruch 1 definierten allgemeinen Formel I herstellt, worin $R^2$ und $R^3$ bzw. $R^1$ und $R^2$ Chlor bedeuten.

11. Verfahren gemäß Anspruch 10, dadurch gekennzeichnet, daß man Verbindungen der in Anspruch 1 definierten allgemeinen Formel I herstellt, worin $R^4$, $R^5$ und $R^8$ alle Wasserstoff und $R^9$ zusammen mit R eine zusätzliche Bindung bedeuten.

12. Verfahren gemäß Anspruch 2, dadurch gekennzeichnet, daß man 2-[[[2-[(2,6-Dichlorphenyl)imino]-1-imidazolidinyl]oxy]methyl]pyridin-1-oxyd herstellt.

13. Verfahren gemäß Anspruch 2, dadurch gekennzeichnet, daß man 2-[[[2-[(2,3-Dichlorphenyl)imino]-1-imidazolidinyl]oxy]methyl]pyridin-1-oxyd herstellt.

14. Verfahren gemäß Anspruch 2, dadurch gekennzeichnet, daß man 2-[[[2-(2,6-Dichlor-N-methylanilino)-2-imidazolin-1-yl]oxy]methyl]pyridin-1-oxyd herstellt.

15. Verfahren gemäß Anspruch 2, dadurch gekennzeichnet, daß man 2-[[[2-[(2,6-Dichlorphenyl)imino]-1-imidazolidinyl]oxy]methyl]-4-methylpyridin-1-oxyd herstellt.

16. Verfahren gemäß Anspruch 2, dadurch gekennzeichnet, daß man 4-[1-[[2-[(2,6-Dichlorphenyl)imino]-1-imidazolidinyl]oxy]äthyl]pyridin-1-oxyd herstellt.

17. Verfahren gemäß Anspruch 2, dadurch gekennzeichnet, daß man 2-[[[2-[(2,6-Dichlorphenyl)imino]-1-imidazolidinyl]oxy]methyl]-5-methylpyridin-1-oxyd herstellt.

18. Verfahren gemäß Anspruch 1, dadurch gekennzeichnet, daß man 2-[[[2-[(2,6-Dichlorphenyl)imino]-1-imidazolidinyl]oxy]methyl]-6-methylpyridin-1-oxyd herstellt.

19. Verfahren gemäß Anspruch 1, dadurch gekennzeichnet, daß man 2-[[[2-[(2,6-Dichlorphenyl)imino]-1-imidazolidinyl]oxy]methyl]-4-pyridinol-1-oxyd herstellt.

**Claims for the Contracting States: BE, CH, DE, FR, GB, IT, LI, LU, NL, SE**

1. Imidazole derivatives of the general formula

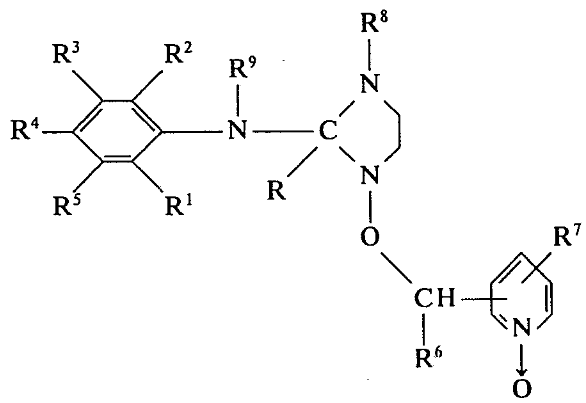

(I)

wherein $R^1$, $R^2$ and $R^3$ independently of one other signify hydrogen, halogen, trifluoromethyl, nitro, lower alkyl or lower alkylthio, one of the residues $R^4$ and $R^5$ signifies hydrogen and the other signifies hydrogen, halogen, hydroxy or lower alkoxy, $R^6$ signifies hydrogen or lower alkyl and $R^7$ signifies hydrogen, hydroxy, lower alkyl or lower alkoxy, and either $R^8$ signifies hydrogen, lower alkyl, lower alkenyl, phenyl-lower alkyl or acyl and $R^9$ together with R signifies an additional bond, or $R^9$ signifies lower alkyl, lower alkenyl or phenyl-lower alkyl and $R^8$ together with R signifies an additional bond, with the proviso that $R^4$ or $R^5$ does not signify hydroxy when $R^7$ signifies lower alkoxy and/or $R^8$ signifies acyl, and that $R^7$ does not signify hydroxy when $R^4$ or $R^5$ signifies lower alkoxy and/or $R^8$ signifies acyl, whereby acyl signifies lower alkanoyl, benzoyl, anisoyl or phenyl-lower alkanoyl and the residues denoted as lower have a maximum of 6 carbon atoms, and pharmaceutically acceptable acid addition salts thereof.

2. Compounds in accordance with claim 1, characterized in that $R^7$ signifies hydrogen, lower alkyl or lower alkoxy.

3. Compounds in accordance with claim 1 or 2, caracterized in that $R^1$, $R^2$ and $R^3$ independently of one another signify hydrogen, fluorine, chlorine, bromine, trifluoromethyl, methyl, ethyl or isopropyl.

4. Compounds in accordance with any one of claims 1 to 3, characterized in that one of the residues $R^4$ and $R^5$ signifies hydrogen and the other signifies hydrogen, chlorine, hydroxy or methoxy

5. Compounds in accordance with any one of claims 1 to 4, characterized in that $R^6$ signifies

hydrogen or methyl.

6. Compounds in accordance with any one of claims 1 and 3 to 5, characterized in that $R^7$ signifies hydrogen, hydroxy, methyl or methoxy.

7. Compounds in accordance with any one of claims 1 to 6, characterized in that $R^8$ signifies hydrogen and $R^9$ together with R signifies an additional bond.

8. Compounds in accordance with any one of claims 1 to 7, characterized in that $R^1$ or $R^3$ signifies hydrogen and $R^2$ and $R^3$ or $R^1$ and $R^2$ have the same significance.

9. Compounds in accordance with claim 8, characterized in that $R^2$ and $R^3$ or $R^1$ and $R^2$ signify halogen.

10. Compounds in accordance with claim 9, characterized in that $R^2$ and $R^3$ or $R^1$ and $R^2$ signify chlorine.

11. Compounds in accordance with claim 10, characterized in that $R^4$, $R^5$ and $R^8$ all signify hydrogen and $R^9$ together with R signifies an additional bond.

12. 2-[[[2-[(2,6-Dichlorophenyl)imino]-1-imidazolidinyl]oxy]methyl]pyridine 1-oxide.

13. 2-[[[2-[(2,3-Dichlorophenyl)imino]-1-imidazolidinyl]oxy]methyl]pyridine 1-oxide.

14. 2-[[[2-(2,6-Dichloro-N-methylanilino)-2-imidazolin-1-yl]oxy]methyl]pyridine 1-oxide.

15. 2-[[[2-[(2,6-Dichlorophenyl)imino]-1-imidazolidinyl]oxy]methyl]-4-methylpyridine 1-oxide.

16. 4-[1-[[2-[(2,6-Dichlorophenyl)imino]-1-imidazolidinyl]oxy]ethyl]pyridine 1-oxide.

17. 2-[[[2-[(2,6-Dichlorophenyl)imino]-1-imidazolidinyl]oxy]methyl]-5-methylpyridine 1-oxide.

18. 2-[[[2-[(2,6-Dichlorophenyl)imino]-1-imidazolidinyl]oxy]methyl]-6-methylpyridine 1-oxide.

19. 2-[[[2-[(2,6-Dichlorophenyl)imino]-1-imidazolidinyl]oxy]methyl]-4-pyridinol 1-oxide.

20. Compounds of the general formula

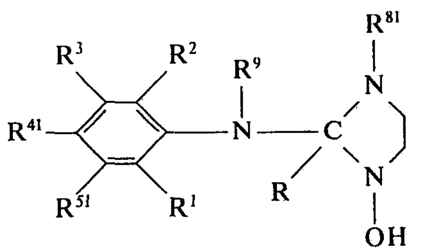

(IIb)

wherein $R^1$, $R^2$ and $R^3$ independently of one another signify hydrogen, halogen, trifluoromethyl, nitro, lower alkyl or lower alkylthio, one of the residues $R^{41}$ and $R^{51}$ signifies hydrogen and the other signifies hydrogen, halogen or lower alkoxy, and either $R^{81}$ signifies lower alkyl, lower alkenyl, phenyl-lower alkyl or acyl and $R^9$ together with R signifies an additional bond, or $R^9$ signifies lower alkyl, lower alkenyl or phenyl-lower alkyl and $R^{81}$ together with R signifies an additional bond, whereby acyl signifies lower alkanoyl, benzoyl, anisoyl or phenyl-lower alkanoyl and the residues denoted as lower have a maximum of 6 carbon atoms.

21. Compounds of the general formula

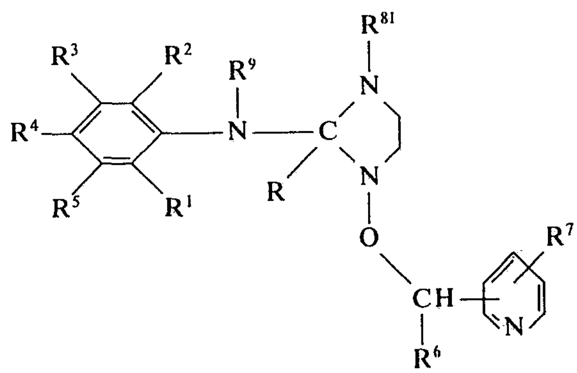

(IV)

wherein $R^1$, $R^2$ and $R^3$ independently of one another signify hydrogen, halogen, trifluoromethyl, nitro, lower alkyl or lower alkylthio, one of the residues $R^4$ and $R^5$ signifies hydrogen and the other signifies hydrogen, halogen, hydroxy or lower alkoxy, $R^6$ signifies hydrogen or lower alkyl and $R^7$ signifies hydrogen, hydroxy, lower alkyl or lower alkoxy, and either $R^{81}$ signifies lower alkyl, lower alkenyl, phenyl-lower alkyl or acyl and $R^9$ together with R signifies an additional bond, or $R^9$ signifies lower alkyl, lower alkenyl or phenyl-lower alkyl and $R^{81}$ together with R signifies an additional bond, with the proviso that $R^4$ or $R^5$ does not signify hydroxy when $R^7$ signifies lower alkoxy and/or $R^8$ signifies acyl, and that $R^7$ does not signify hydroxy when $R^4$ or $R^5$ signifies lower alkoxy and/or $R^8$ signifies acyl, whereby acyl signifies lower alkanoyl, benzoyl, anisoyl or phenyl-lower alkanoyl and the residues denoted as lower have a maximum of 6 carbon atoms.

22. Compounds in accordance with any one of claims 1 to 11 and 13 to 19 as pharmaceutically active substances.

23. Compounds in accordance with any one of claims 1 to 11 and 13 to 19 as analgesically active substances.

24. 2-[[[2-[(2,6-Dichlorophenyl)imino]-1-imidazolidinyl]oxy]methyl]pyridine 1-oxide as a pharmaceutically active substance.

25. 2-[[[2-[(2,6-Dichlorophenyl)imino]-1-imidazolidinyl]oxy]methyl]pyridine 1-oxide as an analgesically active substance.

26. A process for the manufacture of compounds of general formula I defined in claim 1 and their pharmaceutically acceptable acid addition salts, characterized by

a) reacting a compound of the general formula

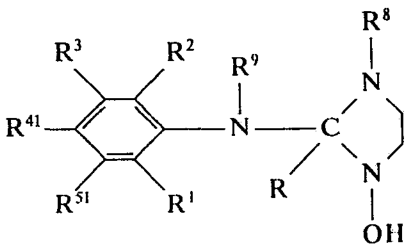

(II)

wherein one of the residues $R^{41}$ and $R^{51}$ signifies hydrogen and the other signifies hydrogen, halogen or lower alkoxy, and $R^1$, $R^2$, $R^3$, $R^8$, $R^9$ and R have the significance given in claim 1, with a compound of the general formula

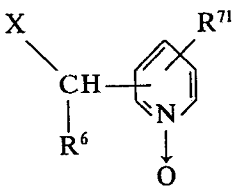

(III)

wherein X signifies a leaving group and $R^{71}$ signifies hydrogen, lower alkyl or lower alkoxy, and $R^6$ has the significance given in claim 1.

b) acylating a compound of the general formula

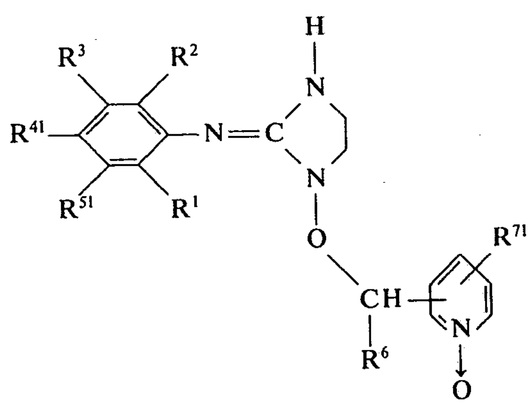

(Ia)

wherein $R^1$, $R^2$, $R^3$ and $R^6$ have the significance given in claim 1 and $R^{41}$, $R^{51}$ and $R^{71}$ have the above significance, at the secondary nitrogen atom, or

c) subjecting a compound of the general formula

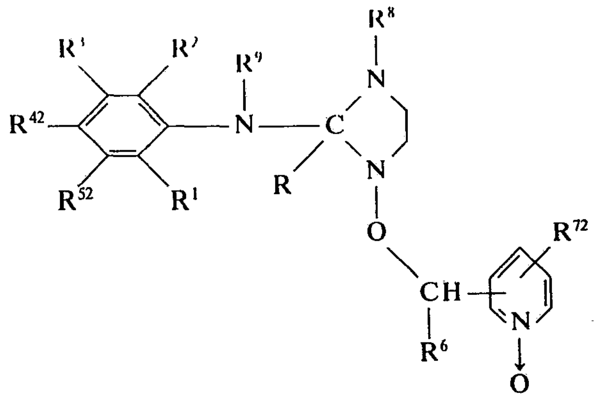

(Ib)

wherein one of the residues $R^{42}$ and $R^{52}$ signifies hydrogen and the other signifies lower alkoxy and $R^{72}$ signifies hydrogen, lower alkyl or lower alkoxy, or $R^{72}$ signifies lower alkoxy and one of the residues $R^{42}$ and $R^{52}$ signifies hydrogen and the other signifies hydrogen, halogen or lower alkoxy, and $R^1$, $R^2$, $R^3$, $R^6$, $R^8$, $R^9$ and R have the significance given in claim 1,
to conditions suitable for ether cleavage, or

d) oxidizing a compound of the general formula

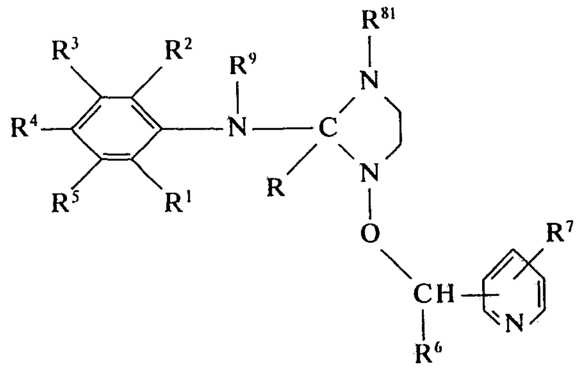

(IV)

wherein either $R^{81}$ signifies lower alkyl, lower alkenyl, phenyl-lower alkyl or acyl and $R^9$ together with R signifies an additional bond, or $R^9$ signifies lower alkyl, lower alkenyl or phenyl-lower alkyl and $R^{81}$ together with R signifies an additional bond, and $R^1$, $R^2$, $R^3$, $R^4$, $R^5$, $R^6$ and $R^7$ have the significance given in claim 1, with the proviso that $R^4$ or $R^5$ does not signify hydroxy when $R^7$ signifies lower alkoxy and/or $R^{81}$ signifies acyl, and that $R^7$ does not signify hydroxy when $R^4$ or $R^5$ signifies lower alkoxy and/or $R^{81}$ signifies acyl,
at the pyridine nitrogen atom, or

e) removing the acyl group in a compound of the general formula

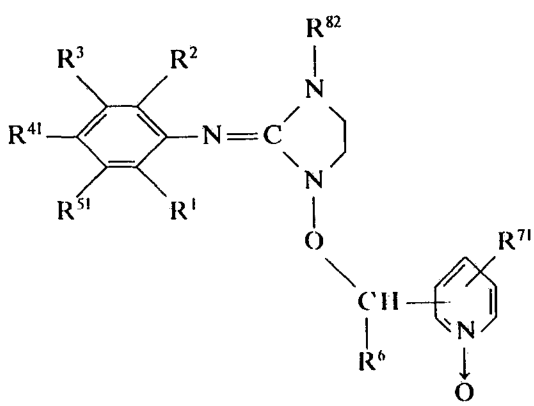

(Ic)

wherein $R^{82}$ signifies acyl, and $R^1$, $R^2$, $R^3$ and $R^6$ have the significance given in claim 1 and $R^{41}$, $R^{51}$ and $R^{71}$ have the above significance,
and

f) if desired, resolving a mixture of optical antipodes obtained, and
g) if desired, converting a compound of general formula I obtained into a pharmaceutically acceptable acid addition salt.

27. A process in accordance with claim 26, characterized in that 2-[[[2-[(2,6-dichlorophenyl)imino]-1-imidazolidinyl]oxy]methyl]pyridine 1-oxide is manufactured.

28. A medicament containing a compound in accordance with any one of claims 1 to 11 and 13 to 19.

29. An analgesic containing a compound in accordance with any one of claims 1 to 11 and 13 to 19.

30. A medicament containing 2-[[[2-[(2,6-dichlorophenyl)imino]-1-imidazolidinyl]oxy]methyl]pyridine 1-oxide.

31. An analgesic containing 2-[[[2-[(2,6-dichlorophenyl)imino]-1-imidazolidinyl]oxy]methyl]pyridine 1-oxide.

**Claims for the Contracting State: AT**

1. A process for the manufacture of imidazole derivatives of the general formula

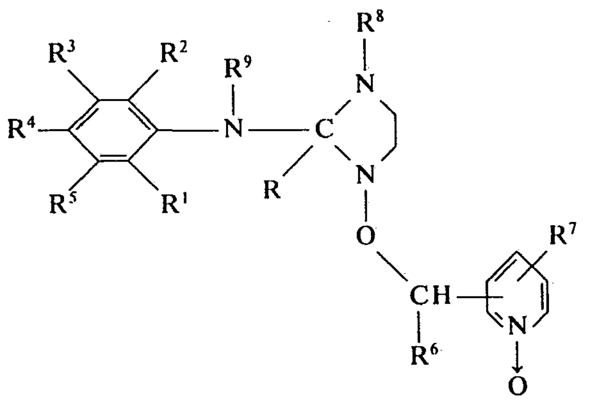

(I)

wherein $R^1$, $R^2$ and $R^3$ independently of one other signify hydrogen, halogen, trifluoromethyl, nitro, lower alkyl or lower alkylthio, one of the residues $R^4$ and $R^5$ signifies hydrogen and the other signifies hydrogen, halogen, hydroxy or lower alkoxy, $R^6$ signifies hydrogen or lower alkyl and $R^7$ signifies hydrogen, hydroxy, lower alkyl or lower alkoxy, and either $R^8$ signifies hydrogen, lower alkyl, lower alkenyl, phenyl-lower alkyl or acyl and $R^9$ together with R signifies an additional bond, or $R^9$ signifies lower alkyl, lower alkenyl or phenyl-lower alkyl and $R^8$ together with R signifies an additional bond, with the proviso that $R^4$ or $R^5$ does not signify hydroxy when $R^7$ signifies lower alkoxy and/or $R^8$ signifies acyl, and that $R^7$ does not signify hydroxy when $R^4$ or $R^5$ signifies lower alkoxy and/or $R^8$ signifies acyl, whereby acyl signifies lower alkanoyl, benzoyl, anisoyl or phenyl-lower alkanoyl and the residues denotes as lower have a maximum of 6 carbon atoms, and of pharmaceutically acceptable acid addition salts thereof, characterized by

a) reacting a compound of the general formula

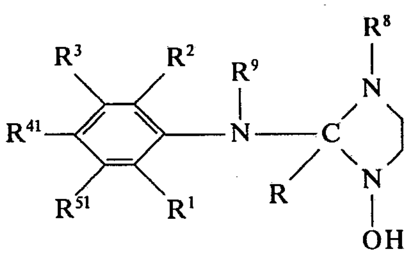

(II)

wherein one of the residues $R^{41}$ and $R^{51}$ signifies hydrogen and the other signifies hydrogen, halogen or lower alkoxy, and $R^1$, $R^2$, $R^3$, $R^8$, $R^9$ and R have the above significance,

with a compound of the general formula

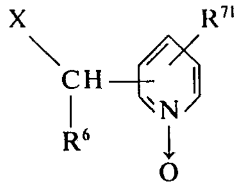

(III)

wherein X signifies a leaving group and $R^{71}$ signifies hydrogen, lower alkyl or lower alkoxy, and $R^6$ has the above significance,
or

b) acylating a compound of the general formula

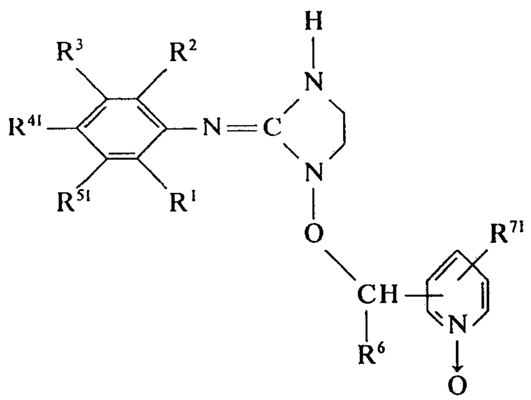

(Ia)

wherein $R^1$, $R^2$, $R^3$, $R^{41}$, $R^{51}$, $R^6$ and $R^{71}$ have the above significance, at the secondary nitrogen atom, or

c) subjecting a compound of the general formula

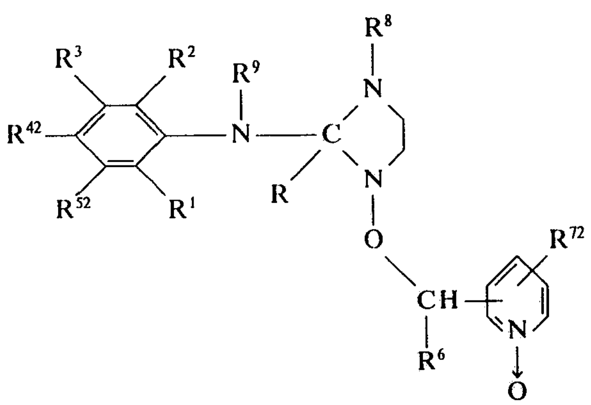

(Ib)

wherein one of the residues $R^{42}$ and $R^{52}$ signifies hydrogen and the other signifies lower alkoxy and $R^{72}$ signifies hydrogen, lower alkyl or lower alkoxy, or $R^{72}$ signifies lower alkoxy and one of the residues $R^{42}$ and $R^{52}$ signifies hydrogen and the other signifies hydrogen, halogen or lower alkoxy, and $R^1$, $R^2$, $R^3$, $R^6$, $R^8$, $R^9$ and R have the above significance,
to conditions suitable for ether cleavage, or

d) oxidizing a compound of the general formula

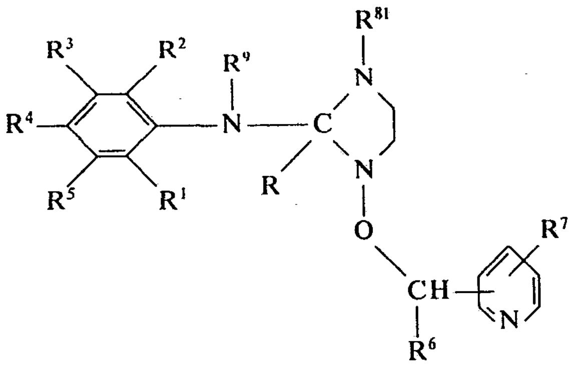

(IV)

wherein either $R^{81}$ signifies lower alkyl, lower alkenyl, phenyl-lower alkyl or acyl and $R^9$ together with R signifies an additional bond, or $R^9$ signifies lower alkyl, lower alkenyl or phenyl-lower alkyl and $R^{81}$ together with R signifies an additional bond, and $R^1$, $R^2$, $R^3$, $R^4$, $R^5$, $R^6$ and $R^7$ have the above significance, with the proviso that $R^4$ or $R^5$ does not signify hydroxy when $R^7$ signifies lower alkoxy and/or $R^{81}$ signifies acyl, and that $R^7$ does not signify hydroxy when $R^4$ or $R^5$ signifies lower alkoxy and/or $R^{81}$ signifies acyl,
at the pyridine nitrogen atom, or

e) removing the acyl group in a compound of the general formula

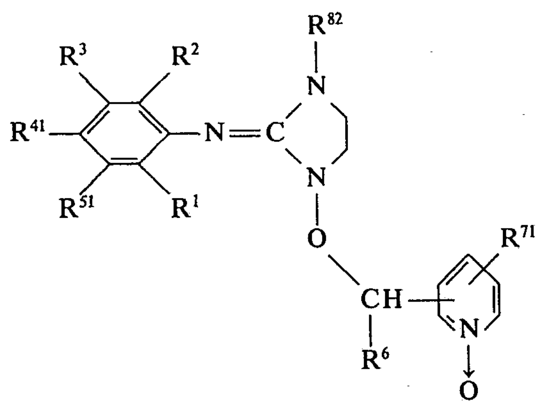

(Ic)

wherein $R^{82}$ signifies acyl, and $R^1$, $R^2$, $R^3$, $R^{41}$, $R^{51}$, and $R^6$ and $R^{71}$ have the above significance, and

f) if desired, resolving a mixture of optical antipodes obtained, and

g) if desired, converting a compound of general formula I obtained into a pharmaceutically acceptable acid addition salt.

2. A process in accordance with claim 1, characterized in that compounds of general formula I defined in claim 1 in which $R^7$ signifies hydrogen, lower alkyl or lower alkoxy are manufactured.

3. A process in accordance with claim 1 or 2, caracterized in that compounds of general formula I defined in claim 1 in which $R^1$, $R^2$ and $R^3$ independently of one another signify hydrogen, fluorine, chlorine, bromine, trifluoromethyl, methyl, ethyl or isopropyl are manufactured.

4. A process in accordance with any one of claims 1 to 3, characterized in that compounds of general formula I defined in claim 1 in which one of the residues $R^4$ and $R^5$ signifies hydrogen and the other signifies hydrogen, chlorine, hydroxy or methoxy are manufactured.

5. A process in accordance with any one of claims 1 to 4, characterized in that compounds of general formula I defined in claim 1 in which $R^6$ signifies hydrogen or methyl are manufactured.

6. A process in accordance with any one of claims 1 and 3 to 5, characterized in that compounds of general formula I defined in claim 1 in which $R^7$ signifies hydrogen, hydroxy, methyl or methoxy are manufactured.

7. A process in accordance with any one of claims 1 to 6, characterized in that compounds of general formula I defined in claim 1 in which $R^8$ signifies hydrogen and $R^9$ together with R signifies an additional bond are manufactured.

8. A process in accordance with any one of claims 1 to 7, characterized in that compounds of general formula I defined in claim 1 in which $R^1$ or $R^3$ signifies hydrogen and $R^2$ and $R^3$ or $R^1$ and $R^2$ have the same significance are manufactured.

9. A process in accordance with claim 8, characterized in that compounds of general formula I defined in claim 1 in which $R^2$ and $R^3$ or $R^1$ and $R^2$ signify halogen are manufactured.

10. A process in accordance with claim 9, characterized in that compounds of general formula I defined in claim 1 in which $R^2$ and $R^3$ or $R^1$ and $R^2$ signify chlorine are manufactured.

11. A process in accordance with claim 10, characterized in that compounds of general formula I defined in claim 1 in which $R^4$, $R^5$ and $R^8$ all signify hydrogen and $R^9$ together with R signifies an additional bond are manufactured.

12. A process in accordance with claim 2, characterized in that 2-[[[2-[(2,6-dichlorophenyl)imino]-1-imidazolidinyl]oxy]methyl]pyridine 1-oxide is manufactured.

13. A process in accordance with claim 2, characterized in that 2-[[[2-[(2,3-dichlorophenyl)imino]-1-imidazolidinyl]oxy]methyl]pyridine 1-oxide is manufactured.

14. A process in accordance with claim 2, characterized in that 2-[[[2-(2,6-dichloro-N-methylanilino)-2-imidazolin-1-yl]oxy]methyl]pyridine 1-oxide is manufactured.

15. A process in accordance with claim 2, characterized in that 2-[[[2-[(2,6-dichlorophenyl)imino]-1-imidazolidinyl]oxy]methyl]-4-methylpyridine 1-oxide is manufactured.

16. A process in accordance with claim 2, characterized in that 4-[1-[[2-[(2,6-dichlorophenyl)imino]-1-imidazolidinyl]oxy]ethyl]pyridine 1-oxide is manufactured.

17. A process in accordance with claim 2, characterized in that 2-[[[(2-[(2,6-dichlorophenyl)imino]-1-imidazolidinyl]oxy]methyl]-5-methylpyridine 1-oxide is manufactured.

18. A process in accordance with claim 1, characterized in that 2-[[[2-[(2,6-dichlorophenyl)imino]-1-imidazolidinyl]oxy]methyl]-6-methylpyridine 1-oxide is manufactured.

19. A process in accordance with claim 1, characterized in that 2-[[[2-[(2,6-dichlorophenyl)imino]-1-imidazolidinyl]oxy]methyl]-4-pyridinol 1-oxide is manufactured.

**Revendications pour les Etats contractants: BE, CH, DE, FR, GB, IT, LI, LU, NL, SE**

1. Dérivés d'imidazoles de formule générale

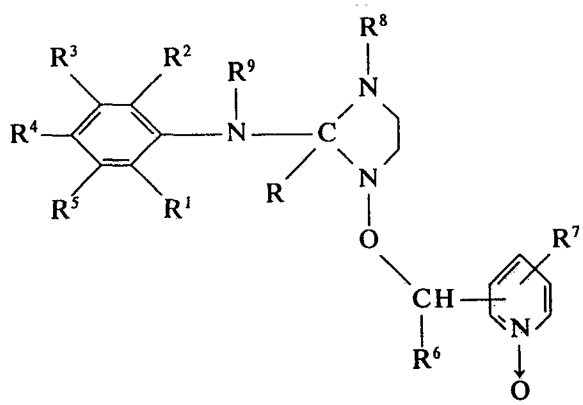

(I)

dans laquelle $R^1$, $R^2$ et $R^3$ représentent chacun, indépendamment l'un de l'autre, l'hydrogène, un halogène, un groupe trifluorométhyle, nitro, alkyle inférieur ou alkylthio inférieur, l'un des symboles $R^4$ et $R^5$ représente l'hydrogène et l'autre l'hydrogène, un halogène, un groupe hydroxy ou alcoxy inférieur, $R^6$ représente l'hydrogène ou un groupe alkyle inférieur et $R^7$ représente l'hydrogène, un groupe hydroxy, alkyle inférieur ou alcoxy inférieur, et ou bien $R^8$ représente l'hydrogène, un groupe alkyle inférieur, alcényle inférieur, phényl-alkyle inférieur ou acyle et $R^9$ représente avec R une liaison supplémentaire, ou bien $R^9$ représente un groupe alkyle inférieur, alcényle inférieur ou phényl-alkyle inférieur et $R^8$ représente avec R une liaison supplémentaire, avec les restrictions que $R^4$ ou $R^5$ ne peut représenter un groupe hydroxy lorsque $R^7$ représente un groupe alcoxy inférieur et/ou $R^8$ un groupe acyle, et que $R^7$ ne peut représenter un groupe hydroxy lorsque $R^4$ ou $R^5$ représente un groupe alcoxy inférieur et/ou $R^8$ un groupe acyle, le groupe acyle consistant en un groupe alcanoyle inférieur, benzoyle, anisoyle ou phényl-alcanoyle inférieur et les restes qualifiés d'inférieurs contenant au maximum 6 atomes de carbone,
et leurs sels formés par addition avec des acides acceptables pour l'usage pharmaceutique.

2. Composés selon la revendication 1, caractérisés en ce que $R^7$ représente l'hydrogène, un groupe alkyle inférieur ou alcoxy inférieur.

3. Composés selon la revendications 1 ou 2, caractérisés en ce que $R^1$, $R^2$ et $R^3$ représentent chacun, indépendamment les uns des autres, l'hydrogène, le fluor, le chlore, le brome, un groupe trifluorométhyle, méthyle, éthyle ou isopropyle.

4. Composés selon l'une des revendications 1 à 3, caractérisés en ce que l'un des symboles $R^4$ et $R^5$ représente l'hydrogène et l'autre l'hydrogène, le chlore, un groupe hydroxy ou méthoxy.

5. Composés selon l'une des revendications 1 à 4, caractérisés en ce que $R^6$ représente l'hydrogène ou un groupe méthyle.

6. Composés selon l'une des revendications 1 et 3 à 5, caractérisés en ce que $R^7$ représente l'hydrogène, un groupe hydroxy, méthyle ou méthoxy.

7. Composés selon l'une des revendications 1 à 6, caractérisés en ce que $R^8$ représente l'hydrogène et $R^9$ représente avec R une liaison supplémentaire.

8. Composés selon l'une des revendications 1 à 7, caractérisés en ce que $R^1$ ou $R^3$ représente l'hydrogène et $R^2$ et $R^3$ d'une part, $R^1$ et $R^2$ d'autre part, ont la même signification.

9. Composés selon la revendication 8, caractérisés en ce que $R^2$ et $R^3$ d'une part, $R^1$ et $R^2$ d'autre part, représentent des halogènes.

10. Composés selon la revendication 9, caractérisés en ce que $R^2$ et $R^3$ d'une part, $R^1$ et $R^2$ d'autre part, représentent le chlore.

11. Composés selon la revendication 10, caractérisés en ce que $R^4$, $R^5$ et $R^8$ représentent tous l'hydrogène et $R^9$ représente avec R une liaison supplémentaire.

12. Le 1-oxyde de 2-[[[2-[(2,6-dichlorophényl)-imino]-1-imidazolidinyl]-oxy]-méthyl]-pyridine.

13. Le 1-oxyde de 2-[[[2-[(2,3-dichlorophényl)-imino]-1-imidazolidinyl]-oxy]-méthyl]-pyridine.

14. Le 1-oxyde de 2-[[[2-(2,6-dichloro-N-méthylanilino)-2-imidazoline-1-yl]-oxy]-méthyl]-pyridine.

15. Le 1-oxyde de 2-[[[2-[(2,6-dichlorophényl)-imino]-1-imidazolidinyl]-oxy]-méthyl]-4-méthyl-pyridine.

16. Le 1-oxyde de 4-[1-[[2-[(2,6-dichlorophényl)-imino]-1-imidazolidinyl]-oxy]-éthyl]-pyridine.

17. Le 1-oxyde de 2-[[[2-[(2,6-dichlorophényl)-imino]-1-imidazolidinyl]-oxy]-méthyl]-5-méthyl-pyridine.

18. Le 1-oxyde de 2-[[[2-[(2,6-dichlorophényl)-imino]-1-imidazolidinyl]-oxy]-méthyl]-6-méthyl-pyridine.

19. Le 1-oxyde de 2-[[[2-[(2,6-dichlorophényl)-imino]-1-imidazolidinyl]-oxy]-méthyl]-4-pyridinol.

20. Composés de formule générale

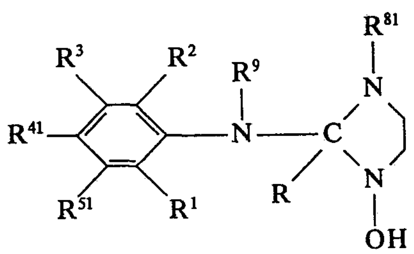

(IIb)

dans laquelle $R^1$, $R^2$ et $R^3$ représentent chacun, indépendamment les uns des autres, l'hydrogène, un halogène, un groupe trifluorométhyle, nitro, alkyle inférieur ou alkylthio inférieur, l'un des symboles $R^{41}$ et $R^{51}$ représente l'hydrogène et l'autre l'hydrogène, un halogène ou un groupe alcoxy inférieur, et ou bien $R^{81}$ représente un groupe alkyle inférieur, alcényle inférieur, phényl-alkyle inférieur ou acyle et $R^9$ représente avec R une liaison supplémentaire, ou bien $R^9$ représente un groupe alkyle inférieur, alcényle inférieur ou phényl-alkyle inférieur et $R^{81}$ représente avec R une liaison supplémentaire, le groupe acyle étant un groupe alcanoyle inférieur, benzoyle, anisoyle ou phényl-alcanoyle inférieur et les restes qualifiés d'inférieurs contenant au maximum 6 atomes de carbone.

21. Composés de formule générale

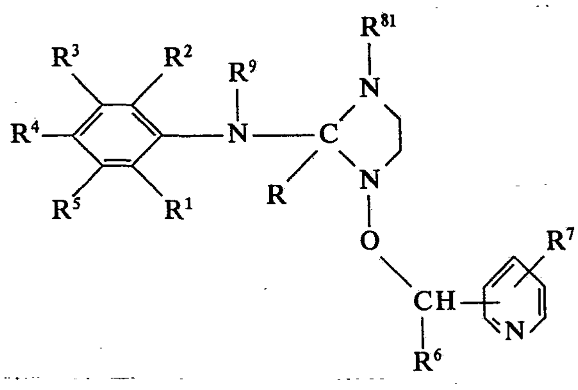

(IV)

dans laquelle $R^1$, $R^2$ et $R^3$ représentent chacun, indépendamment les uns des autres, l'hydrogène, un halogène, un groupe trifluorométhyle, nitro, alkyle inférieur ou alkylthio inférieur, l'un des symboles $R^4$ et $R^5$ représente l'hydrogène et l'autre l'hydrogène, un halogène, un groupe hydroxy ou alcoxy inférieur, $R^6$ représente l'hydrogène ou un groupe alkyle inférieur et $R^7$ représente l'hydrogène, un groupe hydroxy, alkyle inférieur ou alcoxy inférieur, et ou bien $R^{81}$ représente un groupe alkyle

inférieur, alcényle inférieur, phényl-alkyle inférieur ou acyle et $R^9$ représente avec R une liaison supplémentaire, ou bien $R^9$ représente un groupe alkyle inférieur, alcényle inférieur ou phényl-alkyle inférieur et $R^{81}$ représente avec R une liaison supplémentaire, avec les restrictions que $R^4$ ou $R^5$ ne peut représenter un groupe hydroxy lorsque $R^7$ représente un groupe alcoxy inférieur et $R^8$ un groupe acyle, et que $R^7$ ne peut représenter un groupe hydroxy lorsque $R^4$ ou $R^5$ représente un groupe alcoxy inférieur et/ou $R^8$ un groupe acyle, le groupe acyle étant un groupe alcanoyle inférieur, benzoyle, anisoyle ou phényl-alcanoyle inférieur, et les groupes qualifiés d'inférieurs contenant au maximum 6 atomes de carbone.

22. Composés selon l'une des revendications 1 à 11 et 13 à 19 en tant que substances actives pharmaceutiques.

23. Composés selon l'une des revendications 1 à 11 et 13 à 19 en tant que substances actives analgésiques.

24. Le 1-oxyde de 2-[[[2-[(2,6-dichlorophényl)-imino]-1-imidazolidinyl]-oxy]-méthyl]-pyridine en tant que substance active pharmaceutique.

25. Le 1-oxyde de 2-[[[2-[(2,6-dichlorophényl)-imino]-1-imidazolidinyl]-oxy]-méthyl]-pyridine en tant que substance active analgésique.

26. Procédé de préparation des composés de formule générale I de la revendications 1 et de leurs sels formés par addition avec des acides acceptables pour l'usage pharmaceutique, caractérisé en ce que

a) on fait réagir un composé de formule générale

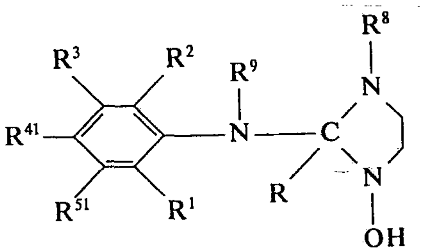

(II)

dans laquelle l'un des symboles $R^{41}$ et $R^{51}$ représente l'hydrogène et l'autre l'hydrogène, un halogène ou un groupe alcoxy inférieur, et $R^1$, $R^2$, $R^3$, $R^8$, $R^9$ et R ont les significations indiquées dans la revendication 1, avec un composé de formule générale

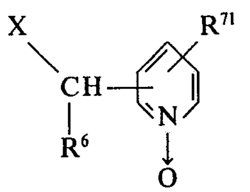

(III)

dans laquelle X représente un groupe éliminable et $R^{71}$ représente l'hydrogène, un groupe alkyle inférieur ou alcoxy inférieur, $R^6$ ayant les significations indiquées dans la revendication 1, ou bien

b) on acyle sur l'atome d'azote secondaire un composé de formule générale

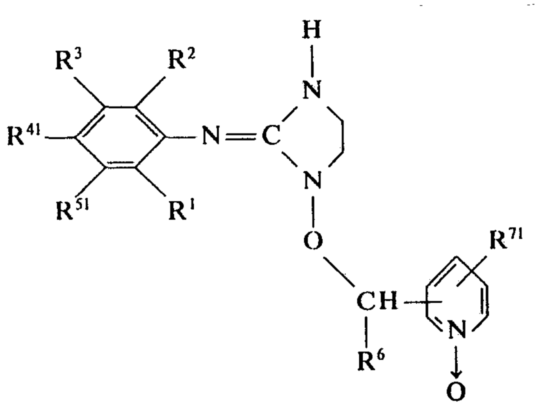

(Ia)

dans laquelle $R^1$, $R^2$, $R^3$ et $R^6$ ont les significations indiquées dans la revendication 1 et $R^{41}$, $R^{51}$ et $R^{71}$ les significations indiquées ci-dessus, ou bien

c) on soumet un composé de formule générale

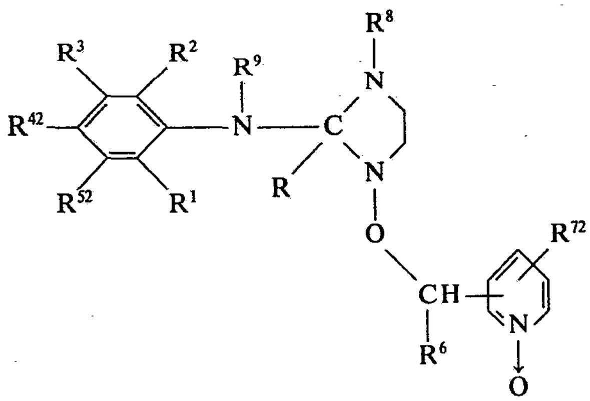

(Ib)

dans laquelle l'un des symboles $R^{42}$ et $R^{52}$ représente l'hydrogène et l'autre un groupe alcoxy inférieur et $R^{72}$ représente l'hydrogène, un groupe alkyle inférieur ou alcoxy inférieur, ou bien $R^{72}$ représente un groupe alcoxy inférieur et l'un des symboles $R^{42}$ et $R^{52}$ représente l'hydrogène et l'autre l'hydrogène, un halogène, un groupe alcoxy inférieur, $R^1$, $R^2$, $R^3$, $R^6$, $R^8$, $R^9$ et R ayant les significations indiquées dans la revendication 1,
à des conditions convenant pour la scission de l'éther, ou bien

d) on oxyde sur l'atome d'azote pyridique un composé de formule générale

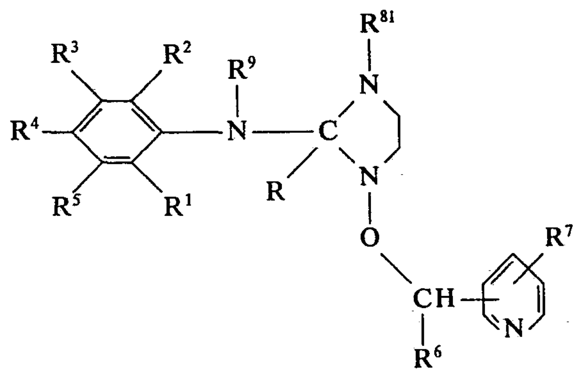

(IV)

dans laquelle ou bien $R^{81}$ représente un groupe alkyle inférieur, alcényle inférieur, phényl-alkyle inférieur ou acyle et $R^9$ représente avec R une liaison supplémentaire, ou bien $R^9$ représente un groupe alkyle inférieur, alcényle inférieur ou phényl-alkyle inférieur et $R^{81}$ représente avec R une liasion supplémentaire, $R^1$, $R^2$, $R^3$, $R^4$, $R^6$ et $R^7$ ayant les significations indiquées dans la revendication 1, avec les restrictions que $R^4$ ou $R^5$ ne peut représenter un groupe hydroxy lorsque $R^7$ représente un groupe alcoxy inférieur et/ou $R^{81}$ un groupe acyle, et que $R^7$ ne peut représenter un groupe hydroxy lorsque $R^4$ ou $R^5$ représente un groupe alcoxy inférieur et/ou $R^{81}$ un groupe acyle, ou bien

e) dans un composé de formule générale

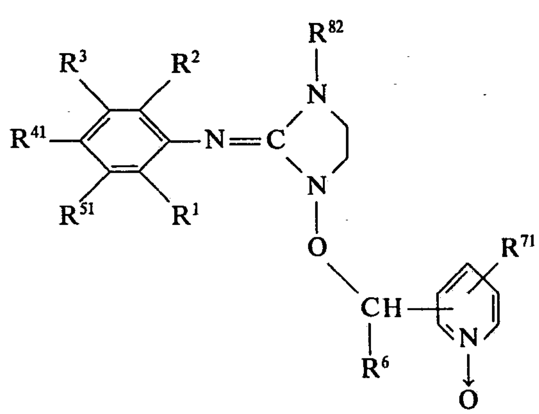

(Ic)

dans laquelle $R^{82}$ représente un groupe acyle, et $R^1$, $R^2$, $R^3$ et $R^6$ ont les significations indiquées

dans la revendication 1 et $R^{41}$, $R^{51}$ et $R^{71}$ les significations indiquées ci-dessus, on élimine le groupe acyle, et

f) si on le désire, on résout un mélange d'antipodes optiques ainsi obtenu, et

g) si on le désire, on convertit un composé obtenu répondant à la formule générale I en un sel par addition avec un acide acceptable pour l'usage pharmaceutique.

27. Procédé selon la revendication 26, caractérisé en ce que l'on prépare le 1-oxyde de 2-[[[2-[(2,6-dichlorophényl)-imino]-1-imidazolidinyl]-oxy]-méthyl]-pyridine.

28. Médicament contenant un composé selon l'une des revendications 1 à 11 et 13 à 19.

29. Analgésique contenant un composé selon l'une des revendications 1 à 11 et 13 à 19.

30. Médicament contenant le 1-oxyde de 2-[[[2-[(2,6-dichlorophényl)-imino]-1-imidazolidinyl]-oxy]-méthyl]-pyridine.

31. Analgésique contenant le 1-oxyde de 2-[[[2-[(2,6-dichlorophényl)-imino]-1-imidazolidinyl]-oxy]-méthyl]-pyridine.

**Revendications pour l'Etat contractant: AT**

1. Procédé de préparation de dérivés d'imidazoles de formule générale

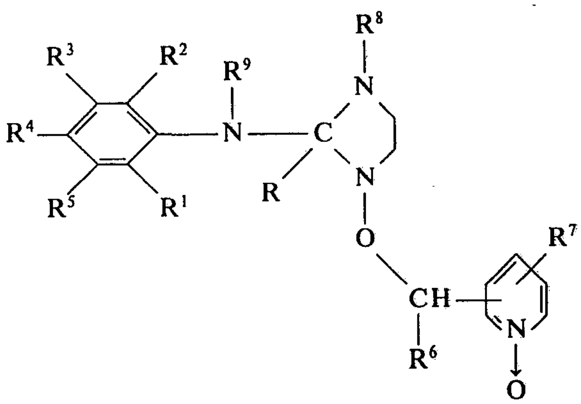

(I)

dans laquelle $R^1$, $R^2$ et $R^3$ représentent chacun, indépendamment les uns des autres, l'hydrogène, un halogène, un groupe trifluorométhyle, nitro, alkyle inférieur ou alkylthio inférieur, l'un des symboles $R^4$ et $R^5$ représente l'hydrogène et l'autre l'hydrogène, un halogène, un groupe hydroxy ou alcoxy inférieur, $R^6$ représente l'hydrogène ou un groupe alkyle inférieur et $R^7$ représente l'hydrogène, un groupe hydroxy, alkyle inférieur ou alcoxy inférieur, et ou bien $R^8$ représente l'hydrogène, un groupe alkyle inférieur, alcényle inférieur, phényl-alkyle inférieur ou acyle et $R^9$ représente avec R une liaison supplémentaire, ou bien $R^9$ représente un groupe alkyle inférieur, alcényle inférieur ou phényl-alkyle inférieur et $R^8$ représente avec R une liaison supplémentaire, avec les restrictions que $R^4$ ou $R^5$ ne peut représenter un groupe hydroxy lorsque $R^7$ représente un groupe alcoxy inférieur et/ou $R^8$ un groupe acyle, et que $R^7$ ne peut représenter un groupe hydroxy lorsque $R^4$ ou $R^5$ représente un groupe alcoxy inférieur et/ou $R^8$ un groupe acyle, le groupe acyle étant un groupe alcanoyle inférieur, benzoyle, anisoyle ou phényl-alcanoyle inférieur et les groupes qualifiés d'inférieurs contenant au maximum 6 atomes de carbone, caractérisés en ce que

a) on fait réagir un composé de formule générale

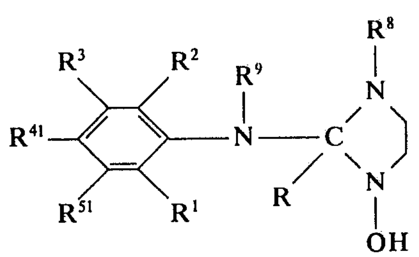

(II)

dans laquelle l'un des symboles $R^{41}$ et $R^{51}$ représente l'hydrogène et l'autre l'hydrogène, un halogène ou un groupe alcoxy inférieur, et $R^1$, $R^2$, $R^3$, $R^8$, $R^9$ et R ont les significations indiquées ci-dessus,

avec un composé de formule générale

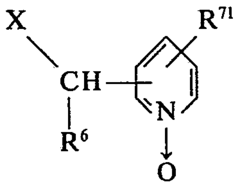

(III)

dans laquelle X représente un groupe éliminable et $R^{71}$ représente l'hydrogène, un groupe alkyle inférieur ou alcoxy inférieur, $R^6$ ayant les significations indiquées ci-dessus, ou bien

b) on acyle sur l'atome d'azote secondaire un composé de formule générale

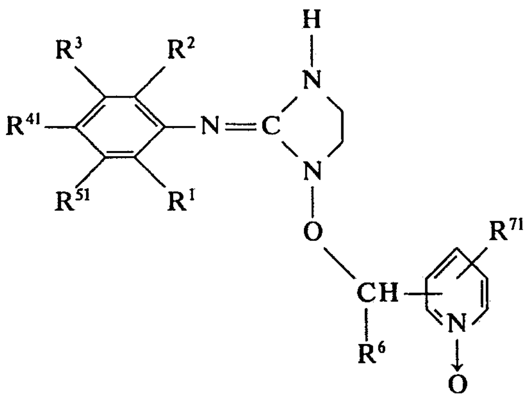

(Ia)

dans laquelle $R^1$, $R^2$, $R^3$, $R^{41}$, $R^{51}$, $R^6$ et $R^{71}$ ont les significations indiquées ci-dessus, ou bien

c) on soumet un composé de formule générale

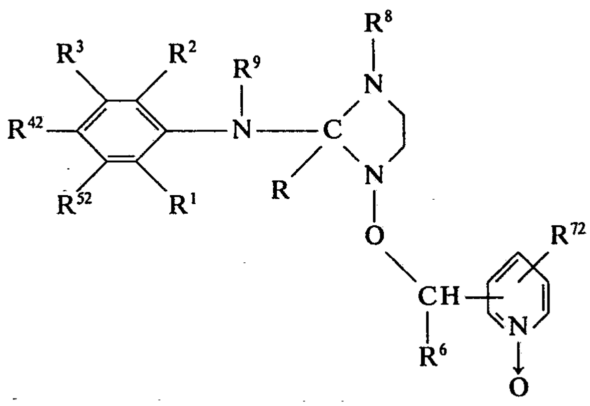

(Ib)

dans laquelle l'un des symboles $R^{42}$ et $R^{52}$ représente l'hydrogène et l'autre un groupe alcoxy inférieur et $R^{72}$ représente l'hydrogène, un groupe alkyle inférieur ou alcoxy inférieur, ou bien $R^{72}$ représente un groupe alcoxy inférieur et l'un des symboles $R^{42}$ et $R^{52}$ représente l'hydrogène et l'autre l'hydrogène, un halogène ou un groupe alcoxy inférieur, $R^1$, $R^2$, $R^3$, $R^6$, $R^8$, $R^9$ et R ayant les significations indiquées ci-dessus,
à des conditions convenant pour la scission de l'éther, ou bien

d) on oxyde sur l'atome d'azote pyridique un composé de formule générale

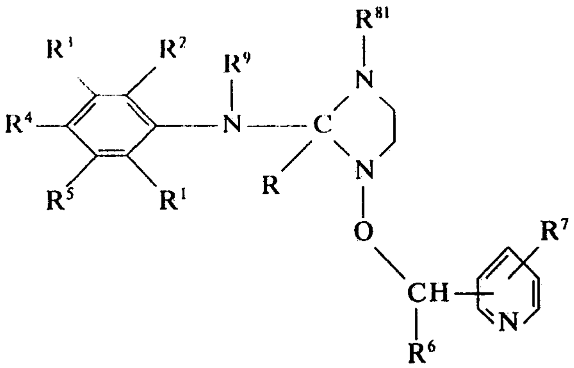

(IV)

dans laquelle ou bien $R^{81}$ représente un groupe alkyle inférieur, alcényle inférieur, phényl-alkyle inférieur ou acyle et $R^9$ représente avec R une liaison supplémentaire, ou bien $R^9$ représente un groupe alkyle inférieur, alcényle inférieur ou phényl-alkyle inférieur et $R^{81}$ représente avec R une liaison supplémentaire, $R^1$, $R^2$, $R^3$, $R^4$, $R^5$, $R^6$ et $R^7$ ayant les significations indiquées ci-dessus, avec les restrictions que $R^4$ ou $R^5$ ne peut représenter un groupe hydroxy lorsque $R^7$ représente un groupe alcoxy inférieur et/ou $R^{81}$ représente un groupe acyle, et que $R^7$ ne peut représenter un groupe hydroxy lorsque $R^4$ ou $R^5$ représente un groupe alcoxy inférieur et/ou $R^{81}$ un groupe acyle, ou bien

e) dans un composé de formule générale

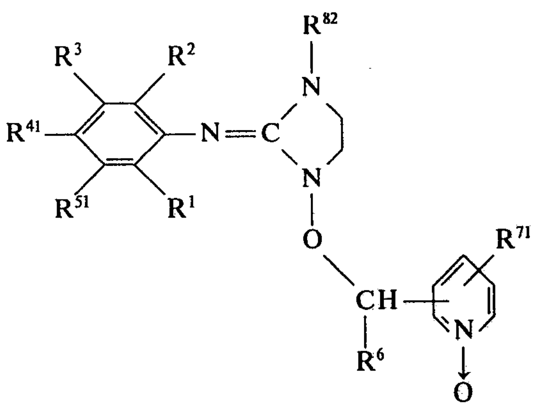

(Ic)

dans laquelle $R^{82}$ représente un groupe acyle, et $R^1$, $R^2$, $R^3$, $R^{41}$, $R^{51}$, $R^6$ et $R^{71}$ ont les significations indiquées ci-dessus,
on élimine le groupe acyle, et

f) si on le désire, on résout un mélange d'antipodes optiques ainsi obtenu, et

g) si on le désire, on convertit un composé obtenu répondant à la formule générale I en un sel par addition avec un acide acceptable pour l'usage pharmaceutique.

2. Composé selon la revendication 1, caractérisé en ce que l'on prépare des composés de formule générale I de la revendication 1 dans laquelle $R^7$ représente l'hydrogène, un groupe alkyle inférieur ou alcoxy inférieur.

3. Procédé selon la revendication 1 ou 2, caractérisé en ce que l'on prépare des composés de formule générale I de la revendication 1 dans laquelle $R^1$, $R^2$ et $R^3$ représentent chacun, indépendamment les uns des autres l'hydrogène, le fluor, le chlore, le brome, un groupe trifluorométhyle, méthyle, éthyle ou isopropyle.

4. Procédé selon l'une des revendications 1 à 3, caractérisé en ce que l'on prépare des composés de formule générale I de la revendication 1 dans laquelle l'un des symboles $R^4$ et $R^5$ représente l'hydrogène et l'autre l'hydrogène, le chlore, un groupe hydroxy ou méthoxy.

5. Procédé selon l'une des revendications 1 à 4, caractérisé en ce que l'on prépare des composés de formule générale I de la revendication 1 dans laquelle $R^6$ représente l'hydrogène ou un groupe méthyle.

6. Procédé selon l'une des revendications 1 et 3 à 5, caractérisé en ce que l'on prépare des composés de formule générale I de la revendication 1 dans laquelle $R^7$ représente l'hydrogène, un groupe hydroxy, méthyle ou méthoxy.

7. Procédé selon l'une des revendications 1 à 6, caractérisé en ce que l'on prépare des composés de

formule I de la revendication 1 dans laquelle $R^8$ représente l'hydrogène et $R^9$ représente avec R une liaison supplémentaire.

8. Procédé selon l'une des revendications 1 à 7, caractérisé en ce que l'on prépare des composés de formule générale I de la revendication 1 dans laquelle $R^1$ ou $R^3$ représente l'hydrogène et $R^2$ et $R^3$ d'une part, $R^1$ et $R^2$ d'autre part, ont les mêmes significations.

9. Procédé selon la revendication 8, caractérisé en ce que l'on prépare des composés de formule générale I de la revendication 1 dans laquelle $R^2$ et $R^3$ d'une part, $R^1$ et $R^2$ d'autre part, représentent des halogènes.

10. Procédé selon la revendication 9, caractérisé en ce que l'on prépare des composés de formule générale I de la revendication 1 dans laquelle $R^2$ et $R^3$ d'une part, $R^1$ et $R^2$ d'autre part, représentent le chlore.

11. Procédé selon la revendication 10, caractérisé en ce que l'on prépare des composés de formule générale I de la revendication 1 dans laquelle $R^4$, $R^5$ et $R^8$ représentent tous des atomes d'hydrogène et $R^9$ représente avec R une liaison supplémentaire.

12. Procédé selon la revendication 2, caractérisé en ce que l'on prépare le 1-oxyde de 2-[[[2-[(2,6-dichlorophényl)-imino]-1-imidazolidinyl]-oxy]-méthyl]-pyridine.

13. Procédé selon la revendication 2, caractérisé en ce que l'on prépare le 1-oxyde de 2-[[[2-[(2,3-dichlorophényl)-imino]-1-imidazolidinyl]-oxy]-méthyl]-pyridine.

14. Procédé selon la revendication 2, caractérisé en ce que l'on prépare le 1-oxyde de 2-[[[2-(2,6-dichloro-N-méthylanilino)-2-imidazoline-1-yl]-oxy]-méthyl]-pyridine.

15. Procédé selon la revendication 2, caractérisé en ce que l'on prépare le 1-oxyde de 2-[[[2-[(2,6-dichlorophényl)-imino]-1-imidazolidinyl]-oxy]-méthyl]-pyridine.

16. Procédé selon la revendication 2, caractérisé en ce que l'on prépare le 1-oxyde de 4-[1-[[2-[(2,6-dichlorophényl)-imino]-1-imidazolidinyl]-oxy]-éthyl]-pyridine.

17. Procédé selon la revendication 2, caractérisé en ce que l'on prépare le 1-oxyde de 2-[[[2-[(2,6-dichlorophényl)-imino]-1-imidazolidinyl]-oxy]-méthyl]-5-méthyl-pyridine.

18. Procédé selon la revendication 2, caractérisé en ce que l'on prépare le 1-oxyde de 2-[[[2-[(2,6-dichlorophényl)-imino]-1-imidazolidinyl]-oxy]-méthyl]-6-méthyl-pyridine.

19. Procédé selon la revendication 2, caractérisé en ce que l'on prépare le 1-oxyde de 2-[[[2-[(2,6-dichlorophényl)-imino]-1-imidazolidinyl]-oxy]-méthyl]-4-pyridinol.